# EUROPÄISCHE PATENTSCHRIFT

(11) **EP 2 603 319 B1**
(45) Veröffentlichungstag und Bekanntmachung des Hinweises auf die Patenterteilung: **22.10.2014**
(21) Anmeldenummer: 11746221.8
(22) Anmeldetag: 09.08.2011
(51) Int. Cl.: B01J 31/22, C07C 263/00

(54) **VERFAHREN ZUR HERSTELLUNG VON URETHANEN**
PROCESS FOR THE PREPARATION OF URETHANS
PROCEDE POUR LA PREPRATION DES URETHANES

(30) Priorität: 12.08.2010 DE 102010039250
(43) Veröffentlichungstag der Anmeldung: 19.06.2013
(73) Patentinhaber: Bayer Intellectual Property GmbH, 40789 Monheim (DE)
(72) Erfinder: GÄRTNER, Felix, 35041 Marburg (DE); JACOB, Andreas, 35039 Marburg (DE); SUNDERMEYER, Jörg, 35041 Marburg (DE); KLEIN, Stephan, 201507 Shanghai (DE); WERSHOFEN, Stefan, 41065 Mönchengladbach (DE)
(74) Vertreter: BIP Patents
(86) Internationale Anmeldenummer: PCT/EP2011/063718
(87) Internationale Veröffentlichungsnummer: WO 2012/020028

(56) Entgegenhaltungen:
- DE-A1-102008 006 881
- KURUSU, YASUHIKO ET AL: "Die Autooxidation von Cumol in Gegenwart von Chelaten monomerer und polymerer Schiffscher Basen", MAKROMOLEKULARE CHEMIE , 176(11), 3185-200 CODEN: MACEAK; ISSN: 0025-116X, 1975, XP002660906,
- UFLYAND I E ET AL: "COMPARATIVE ANALYSIS OF HOMOGENEOUS AND IMMOBILIZED CATALYSTS FOR ETHYLENE DIMERIZATION BASED ON NICKEL(II) CHELATES", JOURNAL OF MOLECULAR CATALYSIS, LAUSANNE, CH, Bd. 55, Nr. 1 - 03, 1. Januar 1989 (1989-01-01), Seiten 302-310, XP000965521, DOI: 10.1016/0304-5102(89)80264-0
- DEBABRATA CHATTERJEE ET AL: "Homogeneous Catalysis of C-H Bond Activation by a Novel Ruthenium(III)-Complex", REACTION KINETICS AND CATALYSIS LETTERS, SPRINGER SCIENCE+BUSINESS MEDIA, DORDRECHT, NL, Bd. 70, Nr. 1, 1. Mai 2000 (2000-05-01), Seiten 147-151, XP019264924, ISSN: 1588-2837

## Beschreibung

Die Erfindung betrifft ein Verfahren zur Herstellung von Urethanen oder Harnstoffen oder Gemischen aus Urethanen und Harnstoffen durch oxidative Carbonylierung von organischen Aminen in Gegenwart von Kohlenmonoxid, Sauerstoff und eines Katalysators, worin als Katalysator ein Übergangsmetallkomplex enthaltend das Strukturmerkmal

[Mⁿ⁺(O~N~O)²⁻](ⁿ⁻²⁾⁺(L)ₘ(Z⁻)ₙ₋₂

eingesetzt wird, wobei M Co bezeichnet, und das Verfahren unter halogenfreien Reaktionsbedingungen durchgeführt wird. Die Erfindung betrifft ferner die Verwendung von Übergangsmetallkomplexen enthaltend obiges Strukturmerkmal als Katalysatoren in der Herstellung von Urethanen oder Harnstoffen oder Gemischen aus Urethanen und Harnstoffen.

Ein Reaktionsschritt bei der Herstellung von Isocyanaten, Urethanen (die auch als Carbamate bezeichnet werden) und auch Harnstoffen im technischen Maßstab ist die Umsetzung des entsprechenden primären Amins mit Phosgen. Hierbei fallen große Mengen Chlorwasserstoff als Koppelprodukt an, die entsorgt oder in gekoppelten Verfahren relativ aufwändig weiter verwendet werden müssen. Seitens der Industrie besteht deshalb großes Interesse an der Entwicklung nichtgekoppelter Syntheseverfahren, die ohne die Verwendung von Phosgen auskommen und das Koppelprodukt Chlorwasserstoff vermeiden.

Ottmann et al. (US-Patentschrift 3 481 967) beschreiben ein Verfahren zur Herstellung von aromatischen Isocyanaten, bei dem aromatische Nitroverbindungen und Kohlenmonoxyd in Gegenwart von Übergangsmetallkatalysatoren eingesetzt werden können. Diese Übergangsmetallkatalysatoren umfassen Cobaltiodid und Titantetrachlorid. Technisch brauchbare Verfahren müssen sich jedoch durch einen hohen Umsatz und eine Selektivität von möglichst über 90 % auszeichnen, um mit Erfolg einsetzbar zu sein. Diese Anforderungen konnten bisher nur von Verfahren der reduktiven Carbonylierung erfüllt werden, die von aromatischen Nitroverbindungen ausgehen und teure Edelmetallkatalysatoren verwenden (B. K. Nefedov, V. I. Manov-Yuvenskii, S. S. Novikov, Doklady Chem. (Proc. Acad. Sci. USSR), 234, (1977), 347.). Meist ist es schwierig, die Edelmetallkatalysatoren wieder zu gewinnen, so dass diese Verfahren vom wirtschaftlichen Standpunkt gesehen nicht tragbar sind.

In WO 2006 131381 A1 wird die reduktive Carbonylierung von aromatischen Nitroverbindungen in Gegenwart von Alkoholen und sauren Promotoren zur Gewinnung von Urethanen offenbart. Die Notwendigkeit der Verwendung saurer Promotoren ist ein Nachteil. Die reduktive Carbonylierung von Nitroaromaten mit ökonomisch preisgünstigen Cobaltkomplexen verläuft in Abwesenheit von Anilin nur sehr langsam. Es wird keine gute Turn-Over-Frequency (TOF) erzielt. Erst wenn sich Anilin bzw. ein Aminoaromat aus dem Nitroaromaten, CO und Protonen als Zwischenprodukt gebildet hat, nimmt die Reaktionsrate der Konvertierung von Nitroaromat zum Carbamat zu. Nachteil der genannten Patentanmeldung ist, dass die besten Ergebnisse erzielt werden, wenn die Komponenten Anilin und Nitroaromat im molaren Verhältnis 2 : 1 vorliegen. Das Vermischen von aromatischen Aminen und aromatischen Nitroverbindungen ist jedoch nicht immer ohne weiteres möglich. So ist beispielsweise hinlänglich bekannt, dass die thermische Stabilität von Dinitrotoluol in Gegenwart von Aminen abnimmt, was zu Sicherheitsrisiken führen kann.

Deshalb hat sich die industrielle und wissenschaftliche Forschung ganz überwiegend auf die Entwicklung von Verfahren konzentriert, bei denen aromatische Aminoverbindungen in Gegenwart von Kohlenmonoxid und einer organischen, Hydroxylgruppen enthaltenden Verbindung durch oxidative Carbonylierung in Gegenwart von Sauerstoff in N-Arylurethane umgewandelt werden, die in einem weiteren Verfahrensschritt in die entsprechenden N-Arylisocyanate überführt werden können.

Ein Beispiel hierfür ist das US-Patent 4 266 070**,** bei dem aromatische Amine in Gegenwart von Co₂(CO)₈ als Katalysator, einer organischen Hydroxylgruppen enthaltenden Verbindung und einer ungesättigten organischen Komponente umgesetzt werden. Letztere ist notwendig, um hohe Selektivitäten zu gewährleisten. Zudem wird ausdrücklich darauf hingewiesen, dass kein Oxidationsmittel, wie z. B. Sauerstoff, zur Anwendung kommt.

Benedini et al. setzten erstmalig [Co^{II}(Salen)] (Salen = N,N'-Disalicyliden-ethylendiamin) als Katalysator ein, um aromatische oder aliphatische primäre Amine in Gegenwart von Methanol in die entsprechenden Urethane bzw. Harnstoffe zu transformieren (F. Benedini, M. Nali, B. Rindone, S. Tollari, J. Mol. Catal, 1986, 34, 155 - 161; G. Maddinelli, M. Nali, B. Rindone, S. Tollari, J. Mol. Catal, 1987, 39, 71 - 77). Nachteilig an diesem Verfahren sind vor allem die langen Reaktionszeiten von bis zu 48 Stunden wie auch immens hohe Katalysatorkonzentrationen (bis zu 20 Mol-%). Die Selektivitäten sind moderat bis gut, wobei in der Regel ein Produktgemisch der entsprechenden Urethane und Harnstoffe erhalten wird.

In dem US-Patent 5 194 660**,** welches die oxidative Carbonylierung von aromatischen Aminen in Gegenwart von Kohlenmonoxid und einer organischen Hydroxylgruppen enthaltenden Verbindung sowie Sauerstoff als Oxidationsmittel beschreibt, wird der Einsatz von Übergangsmetallkomplexkatalysatoren offenbart, die aus Zentrallmetall und makrozyklischen Liganden bestehen. Letztere sind bevorzugt vierzähnige Liganden aus der Gruppe der Porphyrine, Phthalocyanine oder Schiff'-schen Basen. Bei den Liganden aus der Gruppe der Schiff'-schen Basen wird auch die Sechs-Zähnigkeit (oder noch höher) offenbart (Spalte 7, Z. 9 - 11). Zu den Nachteilen dieses Verfahrens ist der Einsatz großer Mengen an Alkalimetallhalogenid, meist Natriumiodid, als Promotor zu zählen. Das Alkalimetall wird dabei im Überschuss zum eigentlichen Katalysator eingesetzt (siehe Beispiele). Aufgrund der großen Halogenmengen sind kaum zu beherrschende Korrosionsprobleme ein weiterer Schwachpunkt dieses Verfahrens.

Es stellte sich deshalb die Aufgabe, ein Verfahren zur Herstellung von Urethanen und/oder Harnstoffen durch oxidative Carbonylierung der entsprechenden primären Amine in Gegenwart von Sauerstoff zur Verfügung zu stellen, das sich durch hohe Umsätze und Selektivitäten bei Verzicht auf halogenhaltige Promotoren auszeichnet.

Die Aufgabe wurde gelöst durch ein Verfahren zur Herstellung von Urethanen oder Harnstoffen oder Gemischen aus Urethanen und Harnstoffen durch oxidative Carbonylierung von organischen Aminen enthaltend primäre Aminogruppen in Gegenwart von Kohlenmonoxid, Sauerstoff und eines Katalysators, worin
als Katalysator ein Übergangsmetallkomplex, bevorzugt ein einkerniger Übergangsmetallkomplex, enthaltend das Strukturmerkmal [Mⁿ⁺(O~N~O)²⁻]⁽ⁿ⁻²⁾⁺(L)ₘ(Z⁻)ₙ₋₂ eingesetzt wird, wobei
M Co bezeichnet,
n = 2 oder 3 ist;
(O~N~O)²⁻ für einen dreizähnigen, dianionischen, bevorzugt halogenfreien, mit M über zwei Sauerstoffatome und ein Stickstoffatom koordinierenden Chelatliganden steht,
L für einen neutralen Liganden steht;
m = 0, 1, 2 oder 3 ist;
Z⁻ für einen monoanionischen Liganden außer Halogenid steht (siehe weiter unten für Details);
und
das Verfahren unter halogenfreien Reaktionsbedingungen, die weiter unten noch im Detail erläutert werden, durchgeführt wird.

Die Nomenklatur der Gruppen des Periodensystems erfolgt in dieser Anmeldung nach der IUPAC-Empfehlung von 1988 (siehe G. J. Leigh, Nomenclature of Inorganic Chemistry, Blackwell Scientific Publications, London, 1990, S. 282). Zu den Begriffen "Zähnigkeit", "Chelatligand" und "Kernigkeit" von Komplexen vgl. Hollemann, Wiberg, Lehrbuch der Anorganischen Chemie, 101. Auflage,Walter de Gruyter & Co. Berlin, 1995, S.1206, 1208 bis 1211.

Die Erfindung betrifft insbesondere weiterhin ein Verfahren, bei dem der neutrale Ligand L ausgewählt ist aus der Gruppe bestehend aus:
Wasser, Azaaromaten, cyclische oder acyclische Amine, cyclische oder acyclische Carbonsäureamide, cyclische oder acyclische Carbonsäureester, cyclische oder acyclische Ketone, cyclische oder acyclische Ketimine, aliphatische Ether, aliphatische oder aromatische Alkohole, Sulfoxide, cyclische oder acyclische Organocarbonate, Nitrile, wobei halogenfreie Vertreter der genannten Verbindungsklassen bevorzugt sind.

Ferner betrifft die Erfindung ein Verfahren, bei dem der monoanionische Ligand Z- ausgewählt ist aus der Gruppe bestehend aus:
Sulfonaten, bevorzugt Triflat, Tosylat, Mesylat, besonders bevorzugt Tosylat und Mesylat;
Carboxylaten, bevorzugt Acetat, Trifluoracetat, Benzoat, besonders bevorzugt Acetat und Benzoat;
Pseudohalogeniden, bevorzugt Cyanid, Cyanat, Thiocyanat, Azid;
perfluorierten Nichtmetall-Koordinationsverbindungen, bevorzugt [BF₄]⁻ [PF₆]⁻, [SbF₆]⁻;
Anionen anorganischer Säuren, bevorzugt Nitrat, Hydrogensulfat, Fluorosulfat, besonders bevorzugt Nitrat und Hydrogensulfat; und
Methylsulfat,
wobei Tosylat, Mesylat, Acetat, Benzoat, Cyanid, Cyanat, Thiocyanat, Azid, Nitrat, Hydrogensulfat und Methylsulfat ganz besonders bevorzugt sind,

Beispiele für bevorzugte Azaaromaten als neutrale Liganden L sind Pyridin, Chinolin, 1,2-Diazin (Pyridazin), 1,3-Diazin (Pyrimidin), 1,4-Diazin (Pyrazin), 1,3,5-Triazin, Imidazol, N-Methylimidazol, Oxazol, Thiazol, Triazol, N-Methyltriazol.

Beispiele für bevorzugte cyclische bzw. acyclische Amine als neutrale Liganden L sind n-Butylamin, Ethylamin, Diethylamin, Triethylamin, Anilin, N-Methylanilin, N,N-Dimethylanilin, Pyrrolidin, N-Metylpyrrolidin, Piperidin, N-Methyl-piperidin, Morpholin, N-Methylmorpholin. Im Fall von primären Aminen als Ligand L ist L bevorzugt identisch mit dem als Edukt eingesetzten Ausgangsamin, um unerwünschte Reaktionen zu vermeiden. Solche primären Amine sind besonders bevorzugt Anilin und Toluylendiamin.

Beispiele für bevorzugte cyclische bzw. acyclische Carbonsäureamide als neutrale Liganden L sind N,N-Dimethylformamid, N,N-Dimethylacetamid, N,N-Dimethylbenzamid, 1-Methyl-2-pyrrolidon, 1-Methyl-2-piperidon, ε-Caprolactam, 1-Methylcaprolactam.

Beispiele für bevorzugte cyclische bzw. acyclische Carbonsäureester als neutrale Liganden L sind ε-Caprolacton, Ethylacetat, Methylbutylat, Cyclohexylacetat.

Beispiele für bevorzugte cyclische bzw. acyclische Ketimine als neutrale Liganden L sind Verbindungen der allgemeinen Formel R^{I}R^{II}C=NR^{III}, worin R^{I}, R^{II} und R^{III} unabhängig voneinander für zyklische oder azyklische Alkylgruppen, Aryl- oder Heteroarylgruppen oder eine verbrückende Alkylengruppe stehen. Besonders bevorzugt enthalten die Reste R^{I}, R^{II} und R^{III} keine Halogene.

Beispiele für bevorzugte aliphatische Ether als neutrale Liganden L sind Diethylether, Dibutylether, Tetrahydrofuran, 1,2-Dimethoxyethan, 1,4-Dioxan.

Beispiele für bevorzugte aliphatische bzw. aromatische Alkohole als neutrale Liganden L sind Methanol, Ethanol, n-Butanol, 2,2,2-Trifluoroethanol, Phenol. Besonders bevorzugt sind Methanol, Ethanol, n-Butanol und Phenol.

Ein Beispiel für bevorzugte Sulfoxide als neutrale Liganden L ist Dimethylsulfoxid.

Beispiele für bevorzugte cyclische bzw. acyclische Organocarbonate als neutrale Liganden L sind Dimethylcarbonat, Diethylcarbonat, Propylencarbonat.

Beispiele für bevorzugte Nitrile als neutrale Liganden L sind Acetonitril, Proprionitril.

Die Liganden L können in der als Substanz isolierten Katalysatorstufe auch fehlen. In Lösung können die Liganden L Solvensmoleküle sein, die leicht durch die umzusetzenden Ausgangsamin substituierbar sind.

Beispiele für bevorzugte Carboxylate als monoanionische Liganden Z⁻ sind Benzoat, Acetat, Trifluoracetat, Formiat. Besonders bevorzugt sind Benzoat, Acetat und Formiat.

Beispiele für bevorzugte Sulfonate als monoanionische Liganden Z⁻ sind Triflat (CF₃SO₃⁻), Tosylat (CH₃-C₆H₄-SO₃⁻, alle Isomere). Besonders bevorzugt ist Tosylat.

Beispiele für bevorzugte Pseudohalogenide als monoanionische Liganden Z- sind CN⁻, SCN⁻, OCN⁻, N₃⁻.

Beispiele für bevorzugte perfluorierte Nichtmetall-Koordinationsverbindungen als monoanionische Liganden Z⁻ sind [BF₄]⁻ [PF₆]⁻, [SbF₆]⁻.

Die Erfindung betrifft insbesondere weiterhin ein Verfahren, bei dem sich der dianionische Chelatligand (O~N~O)²⁻ durch Deprotonierung aller OH-Funktionen, welche nicht Bestandteil der Substituenten R¹ oder R² sind, von neutralen Chelatliganden ausgewählt aus der Gruppe bestehend aus: 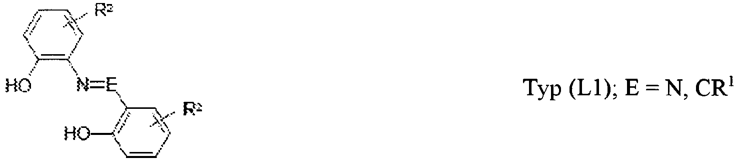 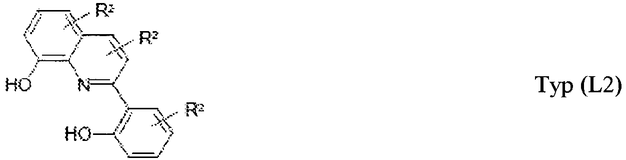 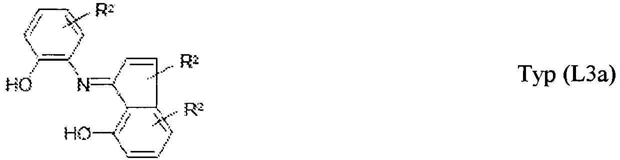 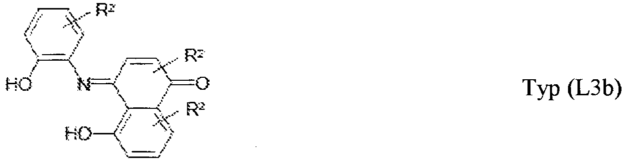 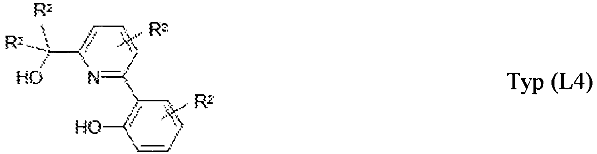 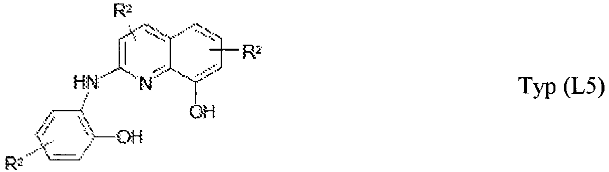 ableitet (für die genauen Definitionen von R¹ und R² wird auf die detaillierten Erläuterungen weiter unten verwiesen).

Besonders bevorzugt sind dabei Ausführungsformen des erfindungsgemäßen Verfahrens, bei denen sich der dianionische Chelatligand (O~N~O)²⁻ durch Deprotonierung aller OH-Funktionen von neutralen Chelatliganden ausgewählt aus der Gruppe bestehend aus: 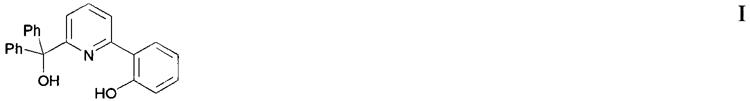 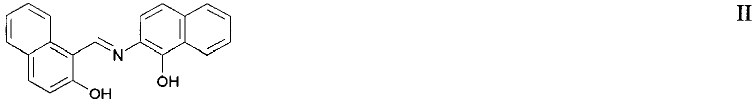 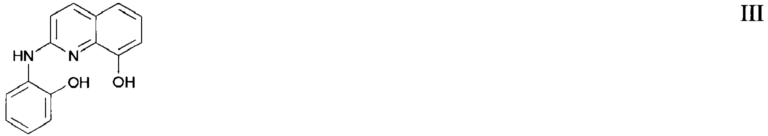 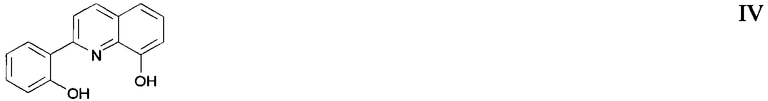 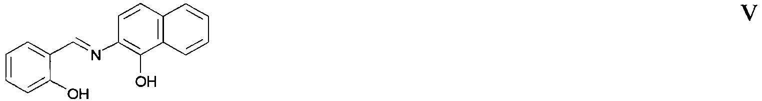 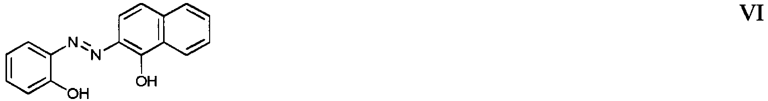 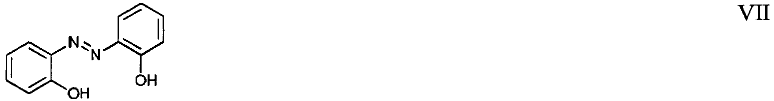 ableitet. Ganz besonders bevorzugt sind dabei die Chelatliganden V und VII, außerordentlich ganz besonders bevorzugt ist VII.

Die Erfindung betrifft insbesondere weiterhin die Verwendung von Übergangsmetallkomplexen, bevorzugt einkernigen Übergangsmetallkomplexen, enthaltend das Strukturmerkmal [Mⁿ⁺(O~N~O)²⁻]⁽ⁿ⁻²⁾⁺(L)ₘ(Z⁻)ₙ₋₂, worin
MCo bezeichnet;
n = 2 oder 3 ist;
(O~N~O)²⁻ für einen dreizähnigen, dianionischen, halogenfreien, mit M über zwei Sauerstoffatome und ein Stickstoffatom koordinierenden Chelatliganden steht, welcher sich durch Deprotonierung aller OH-Funktionen von neutralen Chelatliganden ausgewählt aus der Gruppe bestehend aus: 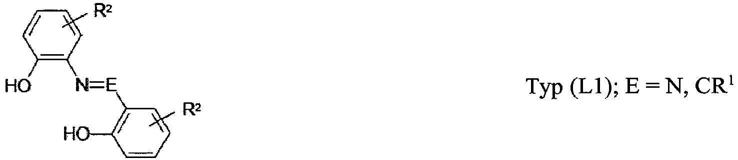 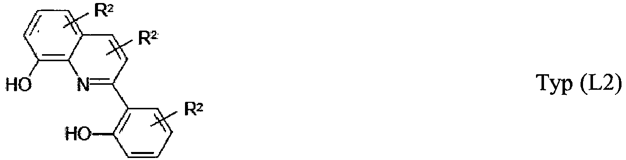 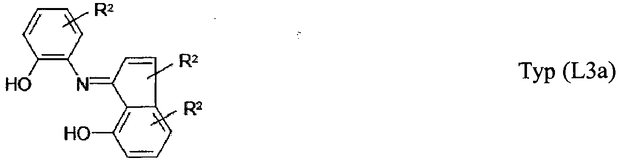 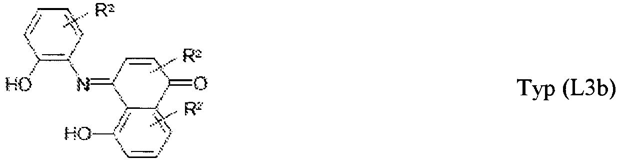 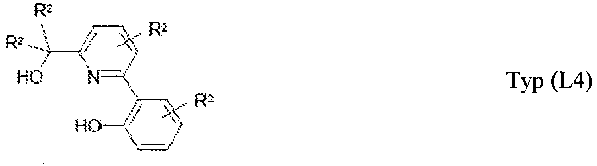 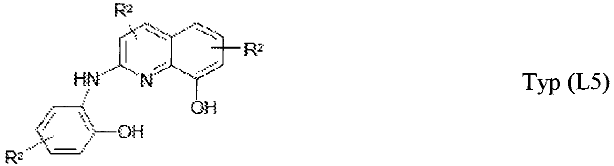 ableitet;
- L: für einen neutralen Liganden ausgewählt aus der Gruppe bestehend aus:
Wasser, Azaaromaten, cyclische oder acyclische Amine, cyclische oder acyclische Carbonsäureamide, cyclische oder acyclische Carbonsäureester, cyclische oder acyclische Ketone, cyclische oder acyclische Ketimine, aliphatische Ether, aliphatische oder aromatische Alkohole, Sulfoxide, Formamide, cyclische oder acyclische Organocarbonate, Nitrile, wobei die halogenfreien Vertreter der genannten Verbindungsklassen bevorzugt sind,
steht;
m = 0, 1, 2 oder 3 ist
und
- Z⁻: für einen monoanionischen Liganden ausgewählt aus der Gruppe bestehend aus:
Sulfonaten, bevorzugt Triflat, Tosylat, Mesylat, besonders bevorzugt Tosylat und Mesylat;
Carboxylaten, bevorzugt Acetat, Trifluoracetat, Benzoat, besonders bevorzugt Acetat und Benzoat;
Pseudohalogeniden, bevorzugt Cyanid, Cyanat, Thiocyanat, Azid;
perfluorierten Nichtmetall-Koordinationsverbindungen, bevorzugt [BF₄]⁻ [PF₆]⁻, [SbF₆]⁻;
Anionen anorganischer Säuren, bevorzugt Nitrat, Hydrogensulfat, Fluorosulfat, besonders bevorzugt Nitrat und Hydrogensulfat; und
Methylsulfat,
wobei Tosylat, Mesylat, Acetat, Benzoat, Cyanid, Cyanat, Thiocyanat, Azid, Nitrat, Hydrogensulfat und Methylsulfat ganz besonders bevorzugt sind,
steht,

als Katalysatoren in Verfahren zur Herstellung von Urethanen oder Harnstoffen oder Gemischen aus Urethanen und Harnstoffen.

In bevorzugten Übergangsmetallkomplexen steht (O~N~O)²⁻ für einen dreizähnigen, dianionischen, mit M über zwei Sauerstoffatome und ein Stickstoffatom koordinierenden Chelatliganden, welcher sich durch Deprotonierung aller OH-Funktionen von neutralen Chelatliganden ausgewählt aus der Gruppe bestehend aus: 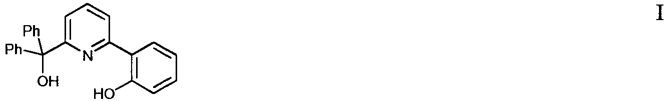 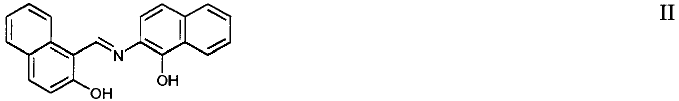 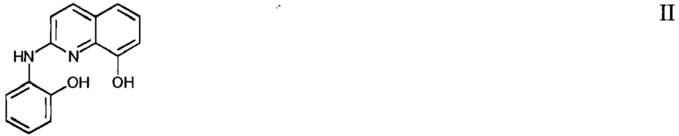 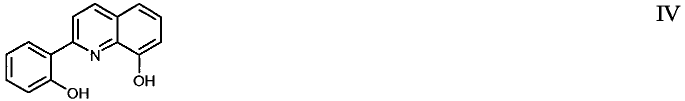 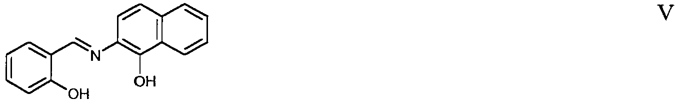 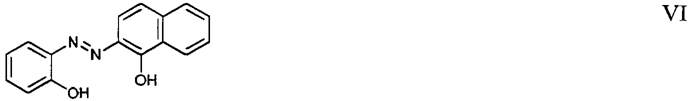 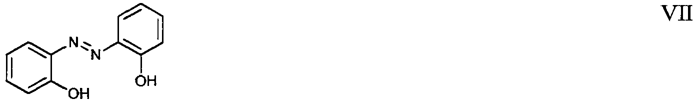 ableitet.
Ganz besonders bevorzugte spezifische Vertreter der einzelnen Bestandteile der Übergangsmetallkomplexe (neutrale Liganden L und monoanionische Liganden Z⁻) sind die gleichen wie bereits zuvor definiert. Daher betrifft die Erfindung ganz besonders bevorzugt die Verwendung von Übergangsmetallkomplexen wie zuvor definiert, bei denen
sich die dianionischen Chelatliganden (O~N~O)²_{~} durch Deprotonierung aller OH-Funktionen von neutralen Chelatliganden ausgewählt aus der Gruppe bestehend aus:
V und VII
ableiten;
die monoanionischen Liganden Z⁻ ausgewählt sind aus der Gruppe bestehend aus
Tosylat, Mesylat, Acetat, Benzoat Cyanid, Cyanat, Thiocyanat, Azid, Nitrat, Hydrogensulfat und Methylsulfat.

Das erfindungsgemäße Verfahren wird unter halogenfreien Reaktionsbedingungen durchgeführt. Dies bedeutet, dass unter den Reaktionsbedingungen Halogene ("Hal") in
(1) elementarer Form (Hal₂ - einschließlich Interhalogenverbindungen - oder Hal) oder
(2) ionischer Form (Hal- oder Polyhalogenide Halₙ⁻ - wie z. B. I₃⁻ -) oder
(3) in Form ihrer Sauerstoffsäuren einschließlich deren Salze
weder zugesetzt noch im Laufe der Reaktion freigesetzt werden.

Dies beinhaltet zum einen, dass auf den Zusatz von halogenhaltigen Promotoren im Sinne von US 5 194 660 vollständig verzichtet wird. Dadurch hebt sich das erfindungsgemäße Verfahren von dieser US-Patentschrift ab, worin 0,05 Massen-% bis 10 Massen-%, bevorzugt 0,1 Massen-%, bis 5 Massen-%, (bezogen auf die eingewogene Menge aller Komponenten) eines halogenhaltigen Promotors eingesetzt werden (Spalte 9, Z. 49 - 53). Unter "halogenhaltigen Promotoren" werden im Rahmen der vorliegenden Erfindung verstanden:
- ionische Metall-Halogenide, bspw. der Alkalimetalle (z. B.: Natriumchlorid, Kaliumchlorid, Natriumbromid, Natriumiodid) oder der Erdalkalimetalle (z. B.: Calciumchlorid, Strontiumbromid, Bariumiodid);
- so genannte "Oniumionen" der allgemeinen Formel [(R^{IV})₁(R^{V})₁(R^{VI})₁(R^{VI})ᵤY]⁺Hal⁻, wobei R^{IV} bis R^{VII} unabhängig voneinander für Wasserstoff, aliphatische bzw. aromatische Reste jeglicher Substitution, Y für die Elemente Stickstoff, Phosphor, Arsen, Antimon, Schwefel, Selen, Telur und Hal für die Halogenide Fluor, Chlor, Brom und Iod stehen und u = 0 oder 1 sein kann;
- Oxo-Säuren (= Sauerstoffsäuren) von Halogenen und ihre Salze (z. B. Perchlorsäure und ihre Salze);
- Komplexverbindungen enthaltend Halogenidionen (Hal⁻);
- organische Halogenverbindungen ("Org-Hal"), bei denen die Kohlenstoff-Halogen-Bindung leicht homolytisch (Org-Hal → Org· + Hal·) oder heterolytisch (Org-Hal → Org⁺+ Hal⁻ - organische "Halogenide") gespalten werden kann als Folge von Resonanzstabilisierung der resultierenden Radikale (Org·) oder Carbokationen (Org⁺), wie z. B. in Ph₃CCl, sowie
- Halogenmoleküle.

Ein Vorteil des Arbeitens ohne halogenhaltige Promotoren liegt - neben der Kostenersparnis bei der Bereitstellung der nötigen Reagenzien - in der Vermeidung der Abtrennung, Rückgewinnung und/oder Rückführung der genannten halogenhaltigen Promotoren. Auch in ökologischer Hinsicht ist der Verzicht auf halogenhaltige Promotoren begrüßenswert.

Dass Halogene im erfindungsgemäßen Verfahren weder zugesetzt noch freigesetzt werden, beinhaltet zum anderen, dass solche Halogenverbindungen, in denen das Grundmolekül über eine starke Bindung mit dem Halogen verknüpft ist, die unter den erfindungsgemäß üblichen Reaktionsbedingungen (siehe weiter unten für Details) stabil ist, sodass keine Halogene, Halogenide oder Halogenwasserstoff freigesetzt werden, nicht als halogenhaltige Promotoren verstanden werden. Der Einsatz solcher Verbindungen bedeutet daher keine Abweichung von den "halogenfreien Reaktionsbedingungen" im Sinne dieser Erfindung. Solche stabilen halogenhaltigen Verbindungen, die nicht als Promotoren einzustufen sind, sind insbesondere
- als Monoanion Z⁻ eingesetzte halogenhaltige Ionen, wie perfluorierte Nichtmetall-Koordinationsverbindungen ([BF₄]⁻ [PF₆]⁻, [SbF₆]⁻), Triflat, Trifluoracetat, Fluorosulfat;
- halogenhaltige Lösungsmittel (wie beispielsweise Dichlormethan, Chloroform, Chlorbenzol, Brombenzol u. a.);
- Alkohole mit Halogensubstitution am Kohlenstoffgrundgerüst (wie beispielsweise Trifluorethanol).

Es ist jedoch in der technischen Praxis denkbar, dass bei der Carbonylierungsreaktion ein bestimmter Gehalt an solchen halogenhaltigen Verbindungen, die prinzipiell unter die oben genannte Definition von halogenhaltigen Promotoren fallen, zwangsläufig vorhanden ist, beispielsweise infolge von Verunreinigungen im ggf. verwendeten Lösungsmittel, in den Edukten, oder stammend aus der Herstellung des Katalysators und/oder des/der Liganden. Solche Spuren an halogenhaltigen Verbindungen, die formal als halogenhaltige Promotoren gelten, sind für das Funktionieren des erfindungsgemäßen Verfahrens nicht erforderlich; sie stören aber im Allgemeinen auch nicht. Daher ist es in der technischen Praxis im Allgemeinen unverhältnismäßig und unökonomisch, durch aufwändige Reinigungsprozesse die Anwesenheit solcher aus Verunreinigungen stammenden Spuren an halogenhaltigen Verbindungen völlig vermeiden zu wollen. Ein solcher Gehalt an aus Verunreinigungen stammenden, nicht bewusst zugesetzten, halogenhaltigen Verbindungen, die formal als halogenhaltige Promotoren gelten, ist im erfindungsgemäßen Verfahren jedoch in jedem Fall geringer als 0,04 Massen-%, bevorzugt geringer als 0,03 Massen-%, besonders bevorzugt geringer als 0,02 Massen-% und ganz besonders bevorzugt geringer als 0,001 Massen-%, bezogen auf die Gesamtmasse des Reaktionsgemisches. Stehen Edukte und Lösungsmittel, die frei von halogenhaltigen Promotoren sind, zu einem wirtschaftlich vertretbaren Preis zur Verfügung, so ist deren Verwendung außerordentlich ganz besonders bevorzugt.

Es wurde gefunden, dass Übergangsmetallkomplexe, die das in Formelschema (i) dargestellte, kleinste gemeinsame Strukturmotiv der (O~N~O)²⁻-Chelatliganden aufweisen, für die oxidative Carbonylierung von organischen Aminen ohne Zusatz von halogenhaltigen Promotoren bevorzugt geeignet sind.

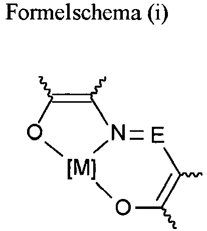

E steht hier und im Folgenden für N oder C-R¹.

R¹ bedeutet im Rahmen dieser Erfindung Wasserstoff, ein Alkylrest mit 1 bis 20 Kohlenstoffatomen, eine Aryl- oder Heteroarylgruppe, eine OR-Gruppe, in der R für Wasserstoff oder eine Alkylgruppe mit 1 bis 20 Kohlenstoffatomen steht, oder eine NRR'-Gruppe, in der R und R' unabhängig voneinander Wasserstoff oder eine Aryl- oder Alkylgruppe mit 1 bis 20 C-Atomen bedeutet oder gemeinsam ein Ringsystem mit Stickstoffatom als Heteroatom bilden können. Bevorzugt steht R¹ für einen Alkylrest mit 1 bis 20 Kohlenstoffatomen, eine Aryl- oder Heteroarylgruppe, eine OR-Gruppe, in der R für Wasserstoff oder eine Alkylgruppe mit 1 bis 20 Kohlenstoffatomen steht, oder eine NRR'-Gruppe, in der R und R' unabhängig voneinander Wasserstoff oder eine Aryl- oder Alkylgruppe mit 1 bis 20 C-Atomen bedeutet oder gemeinsam ein Ringsystem mit Stickstoffatom als Heteroatom bilden können. Hier und im Folgenden sind sämtliche Reste R, R' und Rⁱ (i = arabische Ziffer) bevorzugt halogenfrei.

Generell gilt im Folgenden, dass sämtliche Reste R, R' und Rⁱ (i = arabische Ziffer) einer gegebenen Struktur, wenn sie mehrfach auftreten, gleich sein können aber nicht gleich sein müssen, sondern unabhängig voneinander aus den jeweiligen Definitionsbereichen für die Reste R, R' und Rⁱ (i = arabische Ziffer) ausgewählt sein können.

"~~~" steht für organische Reste, wie sie weiter unten noch näher spezifiziert werden. Auch diese Reste sind hier und im Folgenden bevorzugt halogenfrei.

Die Bezeichnung [M] steht hier und im Folgenden als Platzhalter für zwei- oder dreiwertiges Cobalt und schließt mit ein, dass die Koordinationssphäre am Metall zusätzlich zu dem dianionischen Chelatliganden mit weiteren neutralen Liganden L oder monoanionischen Liganden Z⁻ zu einer Koordinationszahl von 4, 5, 6 oder 7 ergänzt ist, entsprechend dem oben definierten allgemeinen Strukturmerkmal [Mⁿ⁺(O~N~O)²⁻]⁽ⁿ⁻²⁾⁺(L)ₘ(Z⁻)ₙ₋₂. Es bedeutet also: [M] = M²⁺(L)₁ (= Koordinationszahl 4) oder [M] = M²⁺(L)₂ (= Koordinationszahl 5) oder [M] = M²⁺(L)₃ (= Koor-dinationszahl 6) oder [M] = M²⁺(L)₄ (= Koordinationszahl 7) oder [M] = M³⁺(Z⁻)₁ (= Koor-dinationszahl 4) oder [M] = M³⁺(L)₁(Z⁻)₁ (= Koordinationszahl 5) oder [M] = M³⁺(L)₂(Z⁻)₁ (= Koordinationszahl 6) oder [M] = M³⁺(L)₃(Z⁻)₁ (= Koordinationszahl 7). In dieser Nomenklatur beziehen sich die Indices 1 bis 3 bei den neutralen Liganden (L)i auf die Koordinationsstellen dieser Liganden, d. h. ein dreizähniger Ligand ("1 x L~L~L", z. B. Terpyridin) wird genauso als (L)₃ bezeichnet wie drei einzähnige Liganden ("3 x L", z. B. Pyridin).

[M] steht hier und in allen folgenden Formelschemata bevorzugt für Mⁿ⁺(L)ₘ(Z⁻)ₙ₋₂, worin M für Co steht und m und n die zuvor definierten Bedeutungen haben. Der Grund für die Wahl von Cobalt als Zentralmetall ist in seinem geringem Preis und der hohen Aktivität der Cobalt-Komplexe zu sehen.

Der Begriff "Alkyl" umfasst im Sinne der vorliegenden Erfindung azyklische und zyklische gesättigte Kohlenwasserstoffreste, die verzweigt oder geradkettig sowie unsubstituiert oder wenigstens einfach substituiert sein können. Als geeignete Alkyl-Reste, die unsubstituiert oder einfach oder mehrfach substituiert sein können, seien beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, -C(H)(C₂H₅)₂, . -C(H)(n-C₃H₇)₂ und -CH₂-CH₂-C(H)(CH₃)-(CH₂)₃-CH₃ genannt.

Der Begriff "Aryl" bedeutet im Sinne der vorliegenden Erfindungen einen mono- oder polyzyklischen, bevorzugt einen mono- oder bizyklischen, aromatischen Kohlenwasserstoff-Rest mit bevorzugt 6, 10 oder 14 Kohlenstoffatomen. Ein Aryl-Rest kann unsubstituiert oder einfach substituiert oder mehrfach gleich oder verschieden substituiert sein. Als geeignete Aryl-Reste seien beispielsweise Phenyl-, 1-Naphthyl, 2-Naphthyl und Anthracenyl genannt. Besonders bevorzugt ist ein Phenyl-Rest.

Der Begriff "Heteroaryl" bedeutet im Sinne der vorliegenden Erfindung einen monozyklischen oder polyzyklischen, bevorzugt einen mono-, bi- oder trizyklischen, aromatischen Kohlenwasserstoff-Rest mit bevorzugt 5, 6, 7, 8, 9, 10, 11, 12, 13 oder 14 Kohlenstoffatomen, besonders bevorzugt mit 5, 6, 9, 10, 13 oder 14 Kohlenstoffatomen, ganz besonders bevorzugt mit 5 oder 6 Kohlenstoffatomen, in dem ein oder mehrere Kohlenstoffatome jeweils durch ein Heteroatom unabhängig voneinander ausgewählt aus der Gruppe bestehend aus Sauerstoff, Schwefel und Stickstoff (NH) ersetzt wurden. Heteroaryl-Reste können bevorzugt 1, 2, 3, 4 oder 5, besonders bevorzugt 1, 2 oder 3, Heteroatom(e) unabhängig voneinander ausgewählt aus der Gruppe bestehend aus Sauerstoff, Schwefel und Stickstoff (NH) als Ringglied(er) aufweisen. Ein Heteroaryl-Rest kann unsubstituiert oder einfach substituiert oder mehrfach gleich oder verschieden substituiert sein. Als geeignete Heteroaryl-Reste seien beispielsweise Indolizinyl, Benzimidazolyl, Tetrazolyl, Triazinyl, Isoxazolyl, Phthalazinyl, Carbazolyl, Carbolinyl, Diaza-naphthyl, Thienyl, Furyl, Pyrrolyl, Pyrazolyl, Pyrazinyl, Pyranyl, Triazolyl, Pyridinyl, Imidazolyl, Indolyl, Isoindolyl, Benzo[b]furanyl, Benzo[b]thiophenyl, Benzo[d]thiazolyl, Benzodiazolyl, Benzotriazolyl, Benzoxazolyl, Benzisoxazolyl, Thiazolyl, Thiadiazolyl, Oxazolyl, Oxadiazolyl, Isoxazolyl, Pyridazinyl, Pyrimidinyl, Indazolyl, Chinoxalinyl, Chinazolinyl, Chinolinyl, Naphthridinyl und Isochinolinyl genannt.

Aryl- oder Heteroaryl-Reste können im Sinne der vorliegenden Erfindung mit einem mono- bzw. bizyklischem Ringsystem kondensiert (anneliert) sein. Beispielhaft für Aryl-Reste, die mit einem mono- bzw. bizyklischen Ringsystem kondensiert sind, seien (2,3)-Dihydrobenzo[b]thiophenyl, (2,3)-Dihydro-1H-indenyl, Indolinyl, (2,3)-Dihydrobenzofuranyl, (2,3)-Dihydrobenzo[d]oxazolyl, Benzo[d][1,3]dioxolyl, Benzo[d][1,3]oxathiolyl, Isoindolinyl, (1,3)-Diyhydroisobenzofuranyl, (1,3)-Dihydrobenzo[c]thiophenyl, (1,2,3,4)-Tetrahydronaphthyl, (1,2,3,4)-Tetrahydrochinolinyl, Chromanyl, Thiochromanyl, (1,2,3,4)-Tetrahydroisochinolinyl, (1,2,3,4)-Tetrahydrochinoxalinyl, (3,4)-Dihydro-2H-benzo[b][1,4]oxazinyl, (3,4)-Dihydro-2H-benzo[b][1,4]thiazinyl, (2,3)-Dihydro-benzo[b][1,4]dioxinyl, (2,3)-Dihydrobenzo[b][1,4]oxathiinyl, (6,7,8,9)-Tetrahydro-5H-benzo[7]annulenyl, (2,3,4,5)-Tetrahydro-1H-benzo[b]azepinyl und (2,3,4,5)-Tetrahydro-1H-benzo[c]azepinyl genannt.

Sofern einer der oben genannten Reste einfach oder mehrfach substiuiert ist, kommen als Substituenten alle dem Fachmann geläufigen in Betracht, bevorzugt solche, die unabhängig voneinander ausgewählt sind aus der Gruppe bestehend aus F, Cl, Br, I, -NO₂, -CN, -OH, -SH, -NH₂, -N(C₁₋₅-Alkyl)₂, -N(C₁₋₅-Alkyl)(Phenyl), -N(C₁₋₅-Alkyl)(CH₂-Phenyl), -N(C₁₋₅-Alkyl)(CH₂-CH₂-Phenyl), -NH-C(=O)-O-C₁₋₅-Alkyl, -C(=O)-H, -C(=O)-C₁₋₅-Alkyl, -C(=O)-Phenyl, -C(=S)-C₁₋₅-Alkyl, -C(=S)-Phenyl, -C(=O)-OH, -C(=O)-O-C₁₋₅-Alkyl, -C(=O)-O-Phenyl, -C(=O)-NH₂, -C(=O)-NH-C₁₋₅-Alkyl, -C(=O)-N(C₁₋₅-Alkyl)₂, -S(=O)-C₁₋₅-Alkyl, -S(=O)- Phenyl, -S(=O)₂- C₁₋₅-Alkyl, -S(=O)₂-Phenyl, -S(=O)₂-NH₂ und -SO₃H. Besonders bevorzugte Substituenten können unabhängig voneinander ausgewählt werden aus der Gruppe bestehend aus -NO₂, -CN, -OH, -SH, -NH₂, -N(C₁₋₅-Alkyl)₂, -N(C₁₋₅-Alkyl)(Phenyl), -N(C₁₋₅-Alkyl)(CH₂-Phenyl), -N(C₁₋₅-Alkyl)(CH₂-CH₂-Phenyl), -NH-C(=O)-O-C₁₋₅-Alkyl, -C(=O)-H, -C(=O)-C₁₋₅-Alkyl, -C(=O)-Phenyl, -C(=S)-C₁₋₅-Alkyl, -C(=S)-Phenyl, -C(=O)-OH, -C(=O)-O-C₁₋₅-Alkyl, -C(=O)-O-Phenyl, -C(=O)-NH₂, -C(=O)-NH-C₁₋₅-Alkyl, -C(=O)-N(C₁₋₅-Alkyl)₂, -S(=O)-C₁₋₅-Alkyl, -S(=O)- Phenyl, -S(=O)₂- C₁₋₅-Alkyl, -S(=O)₂-Phenyl, -S(=O)₂-NH₂ und -SO₃H. Ganz besonders bevorzugt sind die Substituenten halogenfrei.

Gemeinsames Strukturelement der in Formelschema (i) dargestellten Chelatliganden ist das Vorhandensein einer aromatischen, heteroaromatischen oder vinylischen Baugruppe [-O-C=C-N=], in der eines der beiden O-Ligandhaftatome von dem N-Ligandhaftatom durch zwei sp²-hybridisiedrte Kohlenstoffatome getrennt ist. So ist ein typisches Strukturmotiv solcher Chelatliganden beispielsweise die 1-Amino-2-hydroxy-phenylen-Ligandbaugruppe. Da N,O-Liganden mit diesem Rückgrat selbst Redoxaktivität aufweisen, werden sie als "redox-aktiv" (engl. "redox non-innocent") beschrieben (für den Begriff der "Redoxaktivität" vgl. K. Wieghardt et al., Dalton Transactions, 2003, 1126 - 1132) Die in Formelschema (i) allgemein dargestellten und im Folgenden näher beschriebenen Chelatliganden erfüllen das Kriterium der Ligand-Redoxaktivität.

Es wurde insbesondere gefunden, dass Übergangsmetallkomplexe, die den in Formelschema (ii) dargestellten, speziellen Ausführungsformen des Typs (1) bis (5) (korrespondierend zu den Liganden-Typen (L1) bis (L5)) entsprechen, für die oxidative Carbonylierung von organischen Aminen ohne Zusatz von halogenhaltigen Promotoren besonders bevorzugt geeignet sind.

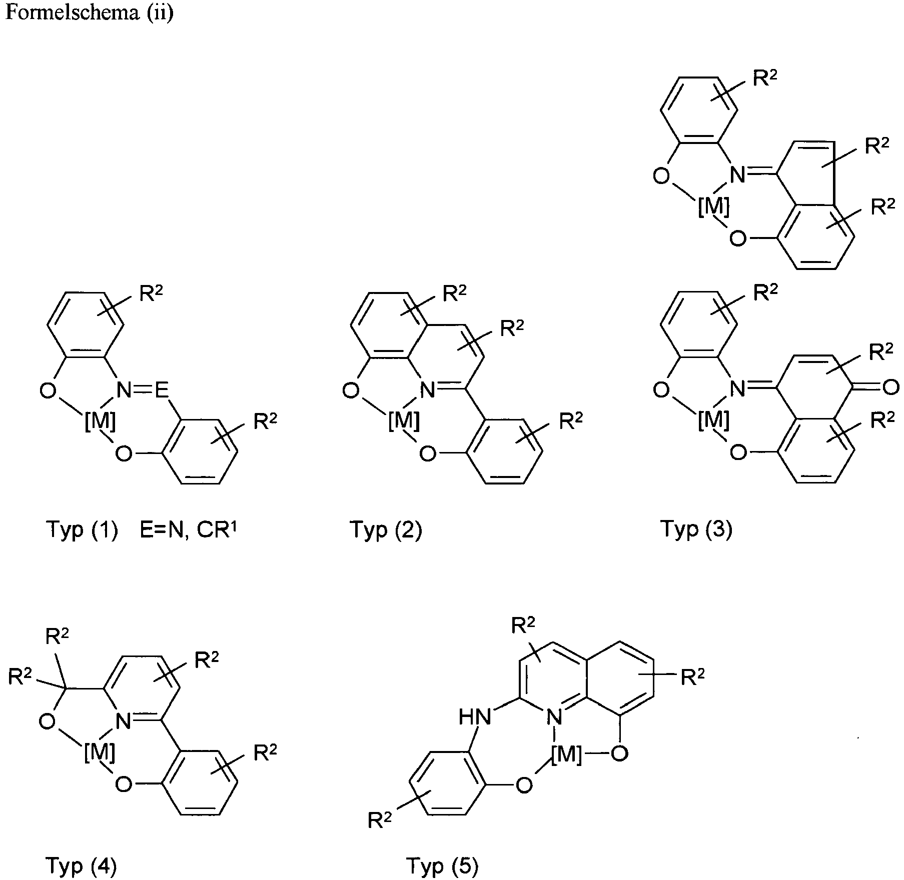

R² bedeutet im Rahmen dieser Erfindung Wasserstoff, eine Alkylgruppe mit 1 bis 20 Kohlenstoffatomen, eine Arylgruppe oder Heteroarylgruppe, wobei die Arylgruppe oder Heteroarylgruppe jeweils entweder über eine Bindung mit dem dreizähnigen Liganden (der bevorzugt einen Salicylat-Baustein enthält) verknüpft oder mit diesem über zwei C-C-Bindungen anelliert ist, eine Ketogruppe -COR, weiterhin -COOR, -COOH, OH, OR oder NRR', wobei R bzw. R' die vorgenannten Bedeutungen haben und R² 1- bis 4-fach den aromatischen Ring substituieren kann.

R³ und R⁴ bedeuten im Rahmen dieser Erfindung unabhängig voneinander Wasserstoff, eine Alkylgruppe mit 1 bis 20 Kohlenstoffatomen, eine Aryl- oder Heteroarylgruppe, eine Ketogruppe - COR, weiterhin -COOR, -COOH, OH, OR oder NRR', wobei R bzw. R' die vorgenannten Bedeutungen haben.

Es wurde weiterhin gefunden, dass Übergangsmetallkomplexe, die eine der in Formelschema (iii) dargestellten Strukturen aufweisen, für die oxidative Carbonylierung von organischen Aminen ohne Zusatz von halogenhaltigen Promotoren ebenfalls besonders bevorzugt geeignet sind.

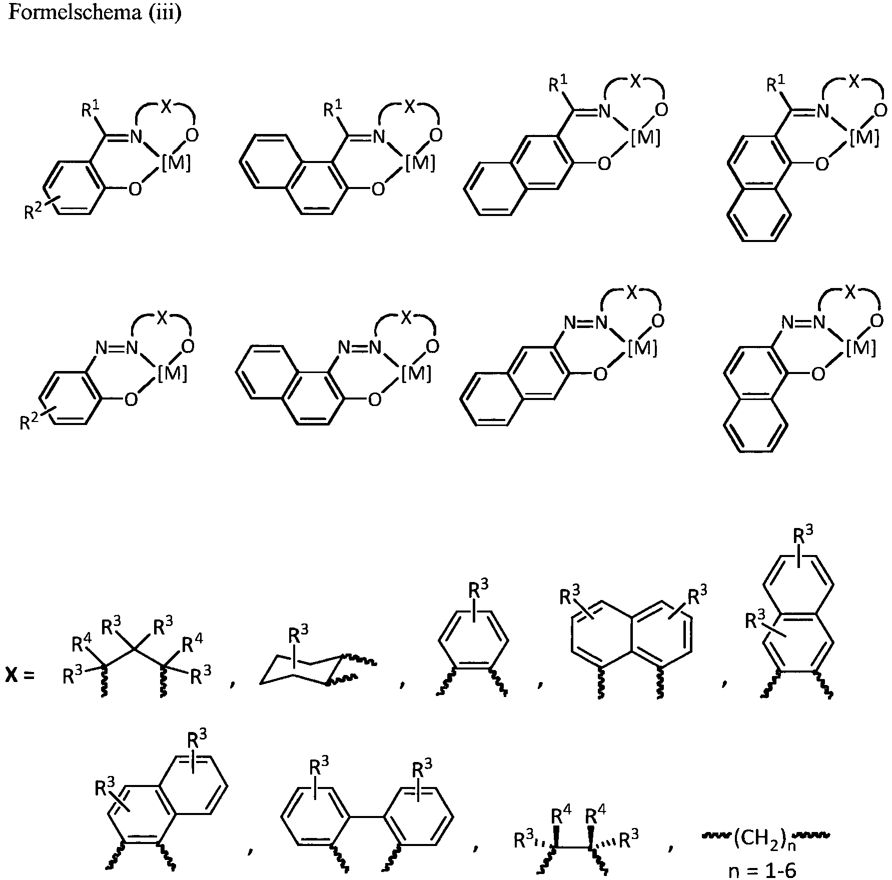

X ist hier und im Folgenden eine beliebige Alkylen- oder Arylen-Baueinheit. Diese Einheit X kann über ihre zentralen Substituenten R³ bzw. R⁴ auch mit weiteren molekularen, makromolekularen oder anorganischen Bauelementen verknüpft sein.

Besonders bevorzugt steht [M] für [Co]. Siehe hierzu auch: R. K. Parashar, R. C. Sharma, A. Kumar, G. Mohan, Inorg. Chim. Acta 1988, 151, 201 - 208; T. Abe, Bull. Chem. Soc. Jpn. 1958, 31, 904 - 907.

Zu den ganz besonders bevorzugten Übergangsmetallkomplexen gemäß den Formelschemata (i) bis (iii) mit (O~N~O)²⁻-Chelatliganden zählen solche, in denen die Chelatliganden drei meridionale Koordinationsstellen des Zentralmetalls Cobalt besetzen, wobei die restlichen drei meridionalen Koordinationsstellen entweder durch die über freie O-Elektronenpaare zusätzlich verbrückende O-Ligandfunktionalität eines benachbarten M(O~N~O) Komplexes im dinuclearen Assoziat oder durch Lösungsmittelmoleküle oder Substratmoleküle mit Donorfunktionalität oder beliebige andere Liganden belegt sind. Unter einer meridionalen (abgekürzt: mer-) Ligandanordnung versteht man, dass die drei koordinierenden Ligandhaftatome und das Metall-Zentralatom näherungsweise in einer Ebene liegen (siehe hierzu auch Joan Ribas Gispert, Coordination Chemistry, Wiley-VCH Weinheim 2008, Chapter 4.3.2 Geometrical Isomers, Seite 102). Außerordentlich besonders bevorzugt sind solche Übergangsmetallkomplexe der Formelschemata (i) bis (iii), in denen die Chelatliganden drei meridionale Koordinationsstellen des Cobalts besetzen.

Ganz besonders bevorzugt von den Verbindungen aus Formelschema (iii) sind die hochaktiven Übergangsmetallkomplexe vom Sap-Typ (Sap = Salicyliden-aminophenol, Formel A), wobei R¹ besonders bevorzugt für H, Methyl (Me) oder Phenyl (Ph) und ganz besonders bevorzugt für Methyl (Me) oder Phenyl (Ph) steht.

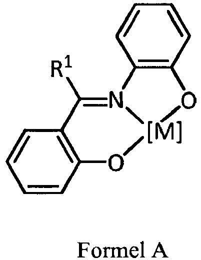

Ebenfalls ganz besonders bevorzugt von den Verbindungen aus Formelschema (iii) ist der Abp-Typ (Abp = Azobisphenol, Formel B).

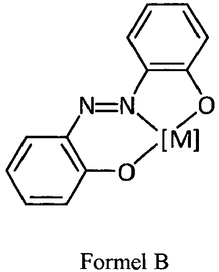

Als ganz besonders geeignet haben sich Komplexe vom Cobalt-Sap- und Cobalt-Abp-Typ ([M] = [Co] in Formel A und B) als Katalysatoren erwiesen. Ihr gemeinsames Strukturelement sind eine Salicylaldehyd- sowie Aminophenol-Baugruppe, die durch ein zusätzliches Atom (Kohlenstoff, Stickstoff) verknüpft sind.

Im erfindungsgemäßen Verfahren einsetzbare Katalysatoren sind auch Übergangsmetallkomplexverbindungen mit anderen dianionischen N₁O₂-Chelatliganden, die Carboxylat-, Azaromat-, Phenolat- und/oder metallierte Carbonsäureamidfunktionen in sich vereinen. Geeignete Liganden mit besonders bevorzugten Strukturelementen sind in den folgenden Formelschemata (iv) und (v), genauer beschrieben:

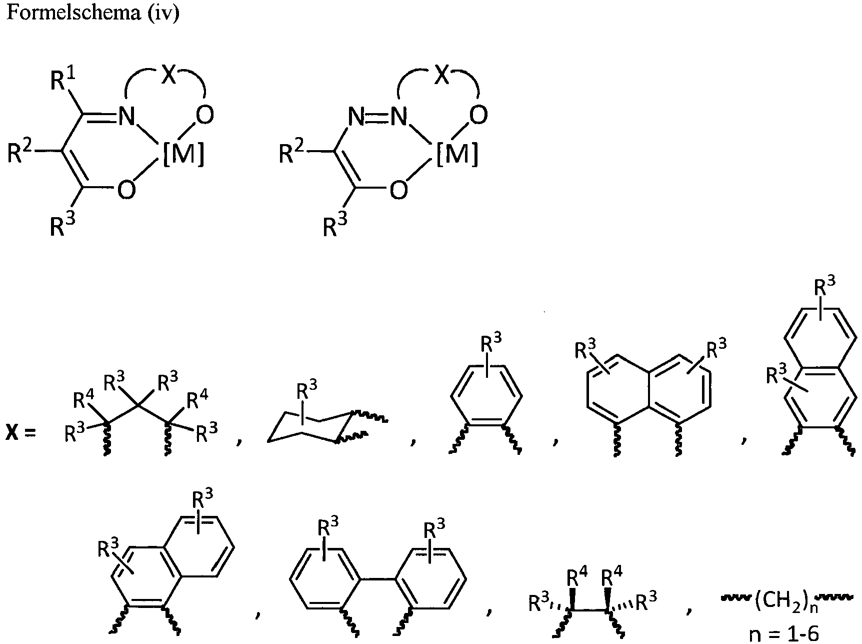

In Formelschema (iv) sind R¹, R², R³, R⁴, X und [M] genauso definiert wie bereits zuvor ausgeführt.

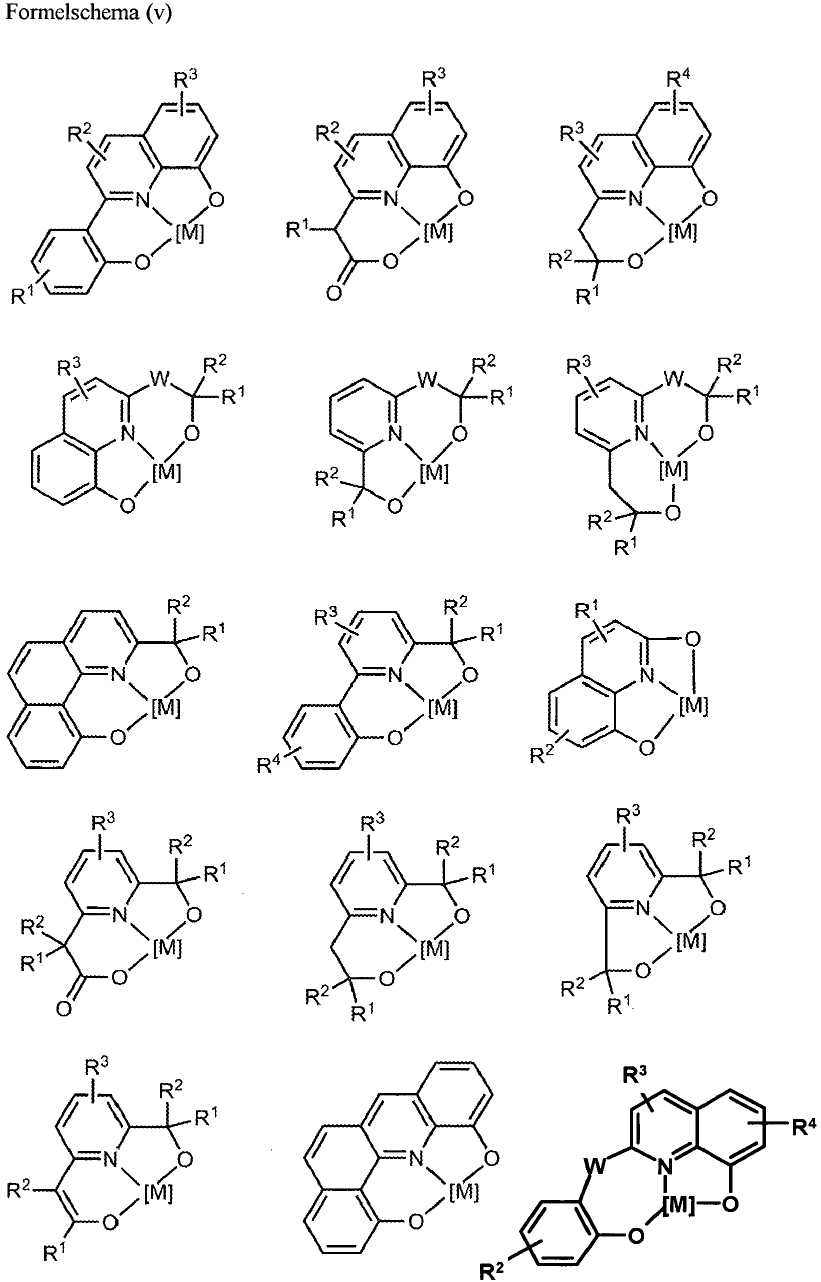

In Formelschema (v) steht W für NH, N-Alkyl, N-Aryl, O, S, CO, CH₂, CHR mit R = C₁-C₂₀-Alkyl, CR₂ oder -Aryl.

In den Formelschemata (iv) und (v) ist [M] wiederum [Co]. Wie in den Formelschemata (i) bis (iii) besetzen auch hier die Chelatliganden bevorzugt drei meridionale Koordinationsstellen des Cobalts, wobei die restlichen drei meridionalen Koordinationsstellen entweder durch die über freie O-Elektronenpaare zusätzlich verbrückende O-Ligandfunktionalität eines benachbarten M(O~N~O) Komplexes im dinuclearen Assoziat oder durch Lösungsmittelmoleküle oder Substratmoleküle mit Donorfunktionalität oder beliebige andere Liganden L belegt sind.

Die Erfindung betrifft demnach insbesondere auch Übergangsmetallkomplexe, bei denen die dreizähnigen, dianionischen, halogenfreien Liganden (O~N~O)²⁻ drei meridionale Koordinationsstellen des Cobalts besetzen.

Mit den vorstehend beschriebenen Katalysatoren lassen sich bei der oxidativen Carbonylierung eines Amins unter halogenfreien Reaktionsbedingungen zu den entsprechenden Harnstoffen Umsätze und Selektivitäten bis zu 99 %, d. h. Ausbeuten bis zu 99 %, bezogen auf das eingesetzte Amin, nach folgender allgemeiner Reaktionsgleichung erzielen:

2 R⁵-NH₂ + CO + 0,5 O₂ → R⁵-NH-CO-NHR⁵ + H₂O (1)

R⁵ ist ein primärer oder sekundärer aliphatischer Rest oder ein aromatischer Rest. Es ist auch möglich, Gemische unterschiedlicher Amine R⁵-NH₂ einzusetzen, so dass der gebildete Harnstoff unsymmetrisch substituiert ist. R⁵-NH₂ steht dabei nicht nur für ein monofunktionelles Amin, sondern auch für ein Aminogruppenäquivalent einer Di- oder Polyaminverbindung (z. B. R⁵ = CH₃-C₆H₃-NH₂, also R⁵-NH₂ = Toluylendiamin). In Di- oder Polyaminverbindungen reagieren bevorzugt alle primären Aminogruppen gemäß Gleichung (1). Unter aliphatischen Resten sind im Rahmen dieser Erfindung sowohl lineare als auch verzweigte als auch cyclische aliphatische Reste zu verstehen.

Bevorzugt wird das erfindungsgemäße Verfahren in Gegenwart organischer, Hydroxylgruppen tragender Verbindungen durchgeführt. Diese Ausführungsform der Erfindung betrifft demnach ein Verfahren, bei dem die oxidative Carbonylierung der organischen Amine in Gegenwart von organischen, Hydroxylgruppen tragenden Verbindungen durchgeführt wird und zur Herstellung von Urethanen oder Gemischen aus Urethanen und Harnstoffen führt. Die Urethanbildung erfolgt dabei gemäß folgender allgemeiner Reaktionsgleichung:

R⁵-NH₂ + CO + 0,5 O₂ + R⁶OH → R⁵-NH-CO-OR⁶ + H₂O (2)

R⁶ ist ein primärer oder sekundärer aliphatischer Rest oder ein aromatischer Rest. R⁵ und R⁶ können auch gleich sein. R⁶ OH steht dabei nicht nur für eine monofunktionelle Hydroxylverbindung, sondern auch für ein Hydroxylgruppenäquivalent einer Di- oder Polyhydroxyverbindung.

Die Reaktionen gemäß der Gleichungen (1) und (2) können auch parallel nebeneinander ablaufen und zu Gemischen aus Urethanen und Harnstoffen führen.

Geeignete organische Amine für das erfindungsgemäße Verfahren zur Herstellung von Urethanen und/oder Harnstoffen sind primäre und sekundäre Amine, wobei primäre Amine (d. h. organische Amine enthaltend primäre Aminogruppen "-NH₂") bevorzugt sind, da sich die daraus erhaltenen Urethane und/oder Harnstoffe in einem weiteren Verfahrensschritt, z. B. durch Thermolyse unter Abspaltung der Hydroxylverbindung R⁶OH bzw. der Aminverbindung R⁵NH₂, in die entsprechenden Isocyanate überführen lassen. Geeignete primäre Amine sind insbesondere aliphatische Mono-, Di- und/oder Polyamine, gemischt aliphatisch-cycloaliphatische Mono-, Di- und/oder Polyamine, cycloaliphatische Mono-, Di- und/oder Polyamine, aromatische Mono-, Di- und/oder Polyamine, araliphatische Mono-, Di- und/oder Polyamine oder Gemische, die zwei oder mehr der vorstehenden primären Amine enthalten. Die Erfindung betrifft also weiterhin ein Verfahren zur oxidativen Carbonylierung von organischen Aminen, bei dem als organische Amine aliphatische, cycloaliphatische, araliphatische und / oder aromatische Mono- oder Diamine eingesetzt werden.

Bevorzugte Beispiele geeigneter primärer Monoamine sind Anilin, am aromatischen Ring einfach oder mehrfach substituierte Aniline wie die isomeren Toluidine oder halogenierte Aniline, Benzylamin, 2-Phenylethylamin, 1-Phenylethylamin, Cyclohexylamin, substituierte Cyclohexylamine, Methylamin, Ethylamin, die isomeren Propyl-, Butyl-, Pentylamine sowie deren höhere Homologe.

Bevorzugte Beispiele geeigneter primärer Diamine sind o-Phenylendiamin, m-Phenylendiamin, p-Phenylendiamin, am aromatischen Ring einfach oder mehrfach substituierte Phenylendiamine wie z. B. Tetramethylphenylendiamin, die isomeren Diaminotoluole wie z. B. 2,4-Diaminotoluol und 2,6-Diaminotoluol, die isomeren Diaminodiphenylmethane wie z. B. 4,4'-Diaminodiphenylmethan, 2,4'-Diaminodiphenylmethan oder 2,2'-Diaminodiphenylmethan, Naphthalindiamine wie z. B. 1,4-Naphthalindiamin, 1,5-Naphthalindiamin oder 1,8-Naphthalindiamin, o-Xylylendiamin, m-Xylylendiamin, p-Xylylendiamin, die isomeren Diaminocyclohexane, am cycloaliphatischen Ring substituierte Diaminocyclohexane, Isophorondiamin, Ethylendiamin, 1,2-Diaminopropan, α,ω-Diaminoalkane wie 1,3-Diaminopropan sowie höhere Homologe wie z. B. 1,6-Hexamethylendiamin, substituierte α,ω-Diaminoalkane.

Gegenstand der Erfindung ist insbesondere ein Verfahren zur oxidativen Carbonylierung von organischen Aminen, bei dem Anilin oder Toluylendiamin, bevorzugt Anilin, als aromatische Amine eingesetzt werden.

Bevorzugte Beispiele geeigneter primärer Polyamine sind durch Kondensation von Anilin mit Formaldehyd erhaltene Gemische der isomeren Diaminodiphenylmethane mit ihren höheren Homologen und deren Isomeren der polyfunktionellen Amine der Diphenylmethanreihe.

Bevorzugte Beispiele organischer, eine oder mehrere Hydroxylgruppen enthaltender Verbindungen sind Alkohole, insbesondere die aliphatischen Alkohole wie Methanol, Ethanol sowie die höheren homologen Alkanole und deren Isomere wie n-Propanol, iso-Propanol, n-Butanol, iso-Butanol, sek-Butanol etc., einfach oder mehrfach Halogen-substituierte Derivate der vorstehenden Alkohole wie z. B. Trifluoroethanol oder Hexafluoroisopropanol, cycloaliphatische Alkohole wie Cylcopentanol oder Cyclohexanol, Benzylalkohol, Phenol oder substituierte Phenole (z. B. Kresole). Besonders bevorzugt betrifft die Erfindung ein Verfahren, bei dem die organischen, Hydroxylgruppen tragenden Verbindungen ausgewählt sind aus der Gruppe bestehend aus:
Methanol, Ethanol, n-Butanol, iso-Butanol, sek-Butanol, n-Propanol, iso-Propanol.

Die Erfindung betrifft weiterhin ein Verfahren, bei dem der Übergangsmetallkomplex-Katalysator enthaltend das Strukturmerkmal [Mⁿ⁺(O~N~O)²⁻]⁽ⁿ⁻²⁾⁺(L)ₘ(Z⁻)ₙ₋₂ in einer Konzentration von 0,01 Mol% bis 10 Mol-%, bevorzugt 0,05 Mol-% bis 5,0 Mol%, besonders bevorzugt 0,1 Mol% bis 3,0 Mol%, bezogen auf ein Mol primärer Aminogruppen, vorliegt.

Bei der oxidativen Carbonylierung eines Amins in Gegenwart einer Hydroxylgruppen enthaltenden Verbindung zu den entsprechenden Urethanen wird die Hydroxylgruppen enthaltende Verbindung in mindestens stöchiometrischer Menge, bezogen auf die Menge an Aminogruppen, eingesetzt. Bevorzugt wird ein Überschuss der Hydroxylgruppen enthaltenden Verbindung, bezogen auf die Menge an Aminogruppen, verwendet. Besonders bevorzugt werden 2 Mol- 100 Mol Hydroxylgruppen pro Mol primärerer Aminogruppen, und ganz besonders bevorzugt 5 Mol- 50 Mol Hydroxylgruppen pro Mol primärerer Aminogruppen eingesetzt.

Im Allgemeinen dient die Hydroxylgruppen enthaltende Verbindung, insbesondere wenn sie im Überschuss eingesetzt wird, als Lösungsmittel. Es ist jedoch auch möglich, ein weiteres Lösungsmittel einzusetzen. Als zusätzliches Lösungsmittel kommen alle Lösungsmittel, die unter den Reaktionsbedingungen inert sind, in Frage. Bevorzugte Beispiele geeigneter Lösungsmittel, sind aliphatische und aromatische Kohlenwasserstoffe und ihre halogenierten Derivate wie z. B. Benzol, Toluol, die isomeren Xylole, Ethylbenzol, Chlorbenzol, die isomeren Dichlorbenzole, Ether, Ester, etc. Besonders bevorzugte Lösungsmittel sind Benzol, Toluol, die isomeren Xylole, Ethylbenzol, halogenfreie Ether und halogenfreie Ester.

In einer weiteren Ausführungsform können auch wasserhaltige Lösungsmittel eingesetzt werden.

Die Umsetzung wird bevorzugt im Temperaturbereich von 20 °C bis 260 °C, besonders bevorzugt bei 80 °C bis 220 °C durchgeführt.

Die Menge an Kohlenmonoxid wird so bemessen, dass mindestens die für die Umsetzung benötigte stöchiometrische Menge, bezogen auf die vorhandenen Aminogruppen, vorhanden ist. Bevorzugt wird mindestens das 10-fache, besonders bevorzugt mindestens das 15-fache der stöchiometrisch benötigten Menge an Kohlenmonoxid eingesetzt. Im Allgemeinen wird dabei maximal das 200-fache der stöchiometrisch benötigten Menge an Kohlenmonoxid eingesetzt. Der absolute Partialdruck des eingesetzten Kohlenmonoxids beträgt bei Raumtemperatur bevorzugt 1,0 bar bis 150 bar, besonders bevorzugt 10 bar bis 100 bar.

Die Menge an Sauerstoff wird so bemessen, dass mindestens die für die Umsetzung benötigte stöchiometrische Menge, bezogen auf die vorhandenen Aminogruppen, vorhanden ist. Bevorzugt wird mindestens das 1,2-fache, besonders bevorzugt mindestens das 1,6-fache der stöchiometrisch benötigten Menge an Sauerstoff eingesetzt. Im Allgemeinen wird dabei maximal das 20-fache der stöichometrisch benötigten Menge an Sauerstoff eingesetzt. Der absolute Partialdruck des eingesetzten Sauerstoffs beträgt bei Raumtemperatur bevorzugt 0,1 bar bis 20 bar, besonders bevorzugt 1,0 bar bis 10 bar. Es wird jedoch in jedem Fall außerhalb der Explosionsgrenzen des Systems CO/O₂ gearbeitet.

Die Erfindung betrifft also weiterhin ein Verfahren, bei dem die oxidative Carbonylierung bei Temperaturen von 20 °C bis 260 °C und bei einem absoluten Kohlenmonoxidpartialdruck von 1,0 bar bis 150 bar sowie bei einem absoluten Sauerstoffpartialdruck von 0,10 bar bis 20 bar durchgeführt wird.

Der Sauerstoff kann entweder als reiner Sauerstoff oder im Gemisch mit unter den Reaktionsbedingungen inerten Gasen, wie z. B. Stickstoff, CO₂, Edelgase etc., bereitgestellt werden. Dabei kann entweder eines dieser inerten Gase oder ein Gemisch aus zwei oder mehr der genannten inerten Gase zum Einsatz kommen. Vorzugsweise werden entweder reiner Sauerstoff oder Luft verwendet.

Bei dem erfindungsgemäßen Verfahren werden durch oxidative Carbonylierung eines Amins in Gegenwart einer Hydroxylgruppen enthaltenden Verbindung die entsprechenden Urethane gebildet. Im Zuge der Reaktion können als Intermediate und/oder Nebenprodukte ggf. auch die auf dem Amin basierenden Harnstoffe auftreten, die im weiteren Verlauf der Umsetzung durch Alkoholyse mit der Hydroxylgruppen enthaltenden Verbindung teilweise oder vollständig zu den entsprechenden Urethanen weiterreagieren können.

Grundsätzlich können die Harnstoffe aber auch durch oxidative Carbonylierung von organischen Aminen in Gegenwart von Kohlenmonoxid und Sauerstoff unter Abwesenheit von organischen, Hydroxylgruppen tragenden Verbindungen gezielt hergestellt werden.

Zur Aufarbeitung des Reaktionsgemisches und zur Isolierung des Produktes können die dem Fachmann bekannten Verfahren oder beliebige Kombinationen der bekannten Verfahren herangezogen werden, z. B. Destillation, Kristallisation, Filtration, Extraktion, Separation mittels Membranverfahren etc. Edukte, Zwischenprodukte, Lösungsmittel und/oder Katalysatoren können zurück gewonnen und wieder an geeigneter Stelle in den Prozess eingespeist werden.

Das Verfahren kann Batch-weise (diskontinuierlich in Chargen), halbkontinuierlich oder kontinuierlich durchgeführt werden.

Das erhaltene Urethan kann bevorzugt in einer weiteren Verfahrensstufe z. B. thermisch oder chemisch in das entsprechende Isocyanat überführt werden. Die Thermolyse kann z. B. katalysiert oder unkatalysiert in Substanz oder in Gegenwart eines geeigneten Lösungsmittels erfolgen. Die Hydroxylgruppen enthaltende Verbindung kann dabei zurückgewonnen und wieder in der Urethansynthese eingesetzt werden. Durch das Schließen des Kreislaufs der Hydroxylgruppen enthaltenden Verbindung kann der Gesamtprozess wirtschaftlich attraktiver gestaltet werden, da nur Verluste an Hydroxylgruppen enthaltender Verbindung durch irreversible Nebenreaktionen ausgeglichen werden müssen. Auch Harnstoffe können, wenngleich unter verschärften Bedingungen, einer solchen Spaltung in das Isocyanat unterworfen werden. Die Verwendung der Urethane ist jedoch bevorzugt.

Die Erfindung betrifft somit auch ein Verfahren zur Herstellung von Isocyanaten durch thermische Spaltung von Urethanen oder Harnstoffen, bei dem Urethane oder Harnstoffe durch oxidative Carbonylierung der entsprechenden Amine nach dem erfindungsgemäßen Verfahren unter Verwendung der erfindungsgemäßen Übergangsmetallkomplexe hergestellt werden und anschließend die Urethane oder Harnstoffe thermisch in die entsprechenden Isocyanate überführt werden.

Bevorzugt erfolgen dabei die Carbonylierung und die Thermolyse in einem Verfahrensschritt.

Das erfindungsgemäße Verfahren wird durch die nachfolgenden Beispiele näher erläutert.

### Beispiele:

### Beispiel 1 (erfindungsgemäß)

In einem 100-cm³-Edelstahlautoklaven mit Polytetrafluorethylenbecher als Insert wurden 0,0592 g (0,22 mmol) **Salicyliden-aminophenol-Cobalt (II)** ([Co^{II}(Sap)], siehe auch Formelschema (vi)), 1,023 g (11,0 mmol) Anilin und 10,692 g (297,2 mmol) Methanol gemischt. Der Autoklav wurde bei Raumtemperatur (ca. 25 °C) mit einem Gemisch aus Sauerstoffgas (4 bar) und Kohlenmonoxidgas (36 bar) befüllt. Nachdem die Gaszufuhr des Autoklaven unterbrochen worden war, wurde der Autoklav in einen Aluminiumheizblock eingesetzt, der auf 200 °C erhitzt war. Innerhalb von 5 Minuten erreichte der Autoklav eine Reaktionstemperatur von 165 °C, die dann konstant gehalten wurde. Nach einer Reaktionsdauer von 3 Stunden wurde der Autoklav durch kaltes Wasser und Eis abgekühlt, bis er nach etwa 5 Minuten Raumtemperatur erreichte. Die Reaktionsmischung wurde dann qualitativ und quantitativ gaschromatographisch untersucht, wobei Naphthalin als interner Standard diente. Der Umsatz an Anilin betrug 100 %, die Ausbeute an Methyl-N-phenylcarbamat 99 %.

### Beispiel 2 (erfindungsgemäß)

Die im Beispiel 1 genannte Reaktion wurde unter ansonsten gleichen Bedingungen mit 0,22 mmol **Azobisphenol-Cobalt (II)** ([Co^{II}(Abp)], siehe auch Formelschema (vi)) als Katalysator wiederholt.

### Beispiel 3 (erfindungsgemäß)

Die im Beispiel 1 genannte Reaktion wurde unter ansonsten gleichen Bedingungen mit 0,22 mmol **Naphthalydenaminophenol-Cobalt (II)** ([Co^{II}(Nap)], siehe auch Formelschema (vi)) als Katalysator wiederholt.

### Beispiel 4 (erfindungsgemäß)

Die im Beispiel 1 genannte Reaktion wurde unter ansonsten gleichen Bedingungen mit 0,22 mmol **Salicylidenaminonapthalenol-Cobalt (II)** ([Co^{II}(San)], siehe auch Formelschema (vi)) als Katalysator wiederholt.

### Beispiel 5 (erfindungsgemäß)

Die im Beispiel 1 genannte Reaktion wurde unter ansonsten gleichen Bedingungen mit 0,22 mmol **Hydroxyphenylquinolinol-Cobalt (II)** ([Co^{II}(Pqo)], siehe auch Formelschema (vi)) als Katalysator wiederholt.

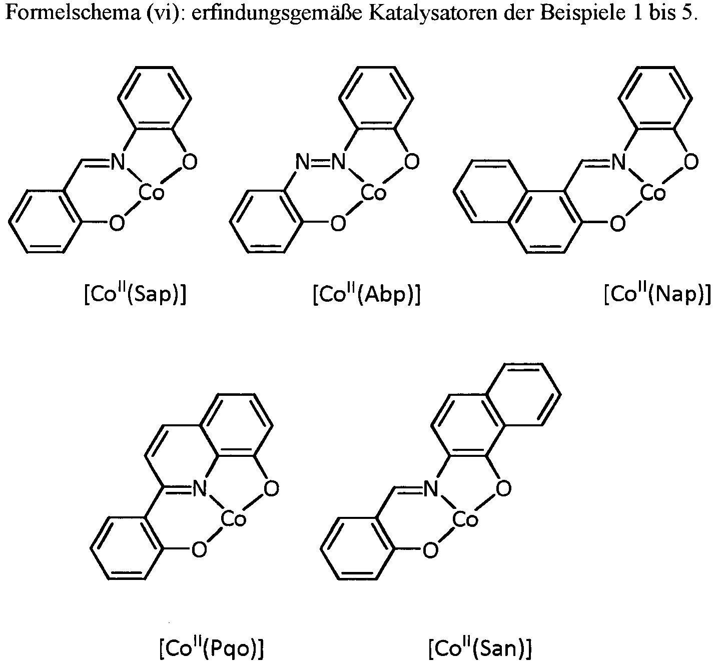

### Beispiel 6 (Vergleichsbeispiel)

Die im Beispiel 1 genannte Reaktion wurde unter ansonsten gleichen Bedingungen ohne Katalysator wiederholt.

### Beispiel 7 (Vergleichsbeispiel)

Die im Beispiel 1 genannte Reaktion wurde unter ansonsten gleichen Bedingungen mit 0,22 mmol **Cobalt(II)-acetat-Tetrahydrat** Co^{II}(OAc)₂ als Katalysator wiederholt.

Die folgende Tabelle fasst die Resultate zusammen:

**Tabelle 1^{[a]}: Ergebnisse der Beispiele 1 bis 8**

| Beispiel Nr. | Katalysator^{[b]} | Umsatz^{[c]} [%] | Selektivität^{[d]} [%] | Ausbeute^{[d]} [%] |
|---|---|---|---|---|
| 1 | [Co^{II}(Sap)] | 100 | 99 | 99 |
| 2 | [Co^{II}(Abp)] | 100 | 93 | 93 |
| 3 | [Co^{II}(Nap)] | 99 | 91 | 90 |
| 4 | [Co^{II}(San)] | 93 | 80 | 74 |
| 5 | [Co^{II}(Pqo)] | 88 | 87 | 77 |
| 6 (Vergleich) | - | 12 | Nicht bestimmt. | Nicht bestimmt. |
| 7 (Vergleich) | Co^{II}(OAc)₂ | 66 | 45 | 30 |

| | | | | |
|---|---|---|---|---|
| [a] Reaktionsbedingungen: 165 °C, 4 bar O₂, 36 bar CO, Reaktionszeit 3 h, molares Verhältnis Katalysator zu Anilin = 1/50, molares Verhältnis Methanol zu Anilin = 27/1. [b] Abp = Azobisphenol, Nap = Naphthalydenaminophenol, Pqo = Hydroxyphenylquinolinol, Sap = Salicylidenaminophenol, San = Salicylidenaminonapthalenol, OAc = Acetat. [c] An Anilin. [d] Bezogen auf Methyl-N-phenylcarbamat. | | | | |

### Beispiel 8 (erfindungsgemäß)

Der Katalysator aus Beispiel 2 wurde in einem molaren Verhältnis von Katalysator zu Anilin von 1/200 unter ansonsten gleichen Bedingungen eingesetzt.

### Beispiel 9 (Vergleichsbeispiel)

Beispiel 8 wurde unter Zusatz von NaI im molaren Verhältnis NaI zu Anilin von 1/10 wiederholt.

Tabelle 2 stellt die Resultate einander gegenüber:

**Tabelle 2^{[a]}: Ergebnisse der Beispiele 8 und 9**

| Beispiel Nr. | Katalysator^{[b]} | Umsatz^{[c]} [%] | Selektivität^{[d]} [%] | Ausbeute^{[d]} [%] |
|---|---|---|---|---|
| 8 | [Co^{II}(Abp)] | 65 | 69 | 45 |
| 9^{[e]} (Vergleich) | [Co^{II}(Abp)] | 63 | 67 | 42 |

| | | | | |
|---|---|---|---|---|
| [a] Reaktionsbedingungen: 165 °C, 4 bar O₂, 36 bar CO, Reaktionszeit 3 h, molares Verhältnis Katalysator zu Anilin = 1/**200**, molares Verhältnis Methanol zu Anilin = 27/1. [b] Abp = Azobisphenol. [c] An Anilin. [d] Bezogen auf Methyl-N-phenylcarbamat. [e] Unter Zusatz von 10 mol-% NaI (bezogen auf Anilin) durchgeführt. | | | | |

Wie man der Tabelle 2 entnehmen kann, führt die Verringerung der Katalysatorbeladung zu einer Verringerung an Ausbeute und Selektivität (vgl. Beispiel 8 mit Beispiel 2). Die erfindungsgemäßen Katalysatoren sind jedoch so leistungsfähig, dass sie selbst unter solchen erschwerten Bedingungen den Einsatz halogenhaltiger Promotoren überflüssig machen (vgl. Beispiel 8 mit Beispiel 9).

## Patentansprüche

1. Verfahren zur Herstellung von Urethanen oder Harnstoffen oder Gemischen aus Urethanen und Harnstoffen durch oxidative Carbonylierung von organischen Aminen enthaltend primäre Aminogruppen in Gegenwart von Kohlenmonoxid, Sauerstoff und eines Katalysators, **dadurch gekennzeichnet, dass**
als Katalysator ein Übergangsmetallkomplex enthaltend das Strukturmerkmal [Mⁿ⁺(O~N~O)¹⁻]⁽ⁿ⁻²⁾⁺(L)ₘ(Z⁻)ₙ₋₂ eingesetzt wird, wobei
M Co bezeichnet,
n = 2 oder 3 ist;
(O~N~O)²⁻ für einen dreizähnigen, dianionischen, mit M über zwei Sauerstoffatome und ein Stickstoffatom koordinierenden Chelatliganden steht,der sich durch Deprotonierung aller OH-Funktionen, welche nicht Bestandteil der Substituenten R¹ oder R² sind, von neutralen Chelatliganden ausgewählt aus der Gruppe bestehend aus: 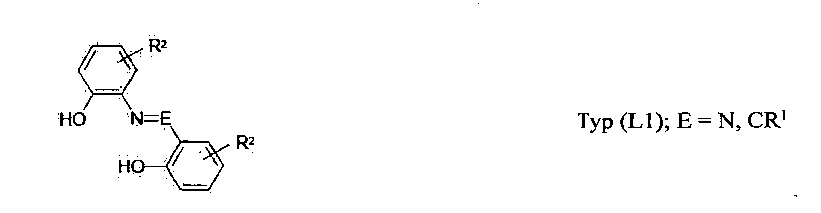 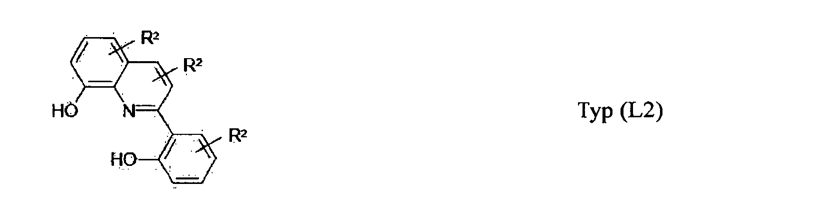 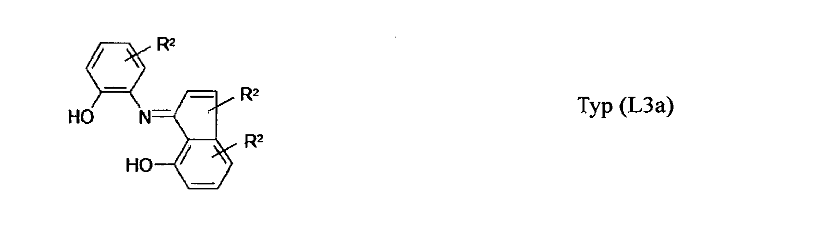 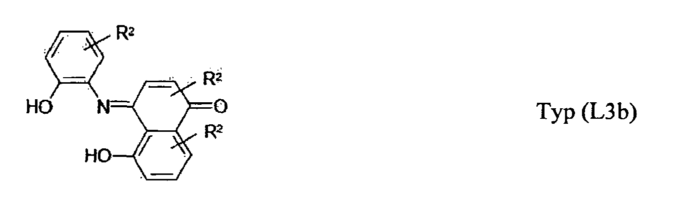 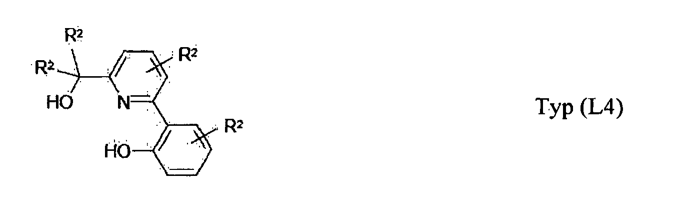 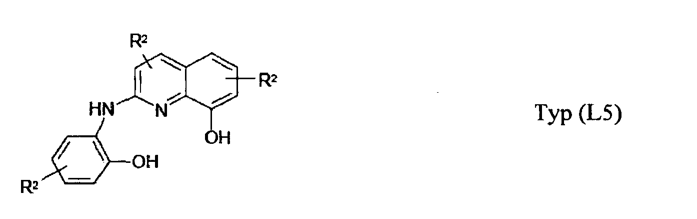 ableitet und worin
R¹ für einen Substituenten ausgewählt aus der Gruppe bestehend aus:
Wasserstoff, eine Alkylgruppe mit 1 bis 20 Kohlenstoffatomen, eine Aryl- oder Heteroarylgruppe, eine OR-Gruppe, in der R für Wasserstoff oder eine Alkylgruppe mit 1 bis 20 Kohlenstoffatomen steht, eine NRR'-Gruppe, in der R und R' unabhängig voneinander Wasserstoff oder eine Aryl- oder Alkylgruppe mit 1 bis 20 C-Atomen bedeuten,
steht;
R² für einen Substituenten ausgewählt der Gruppe bestehend aus:
Wasserstoff, eine Alkylgruppe mit 1 bis 20 Kohlenstoffatomen, eine Aryl-gruppe oder Heteroarylgruppe, wobei die Arylgruppe oder Heteroarylgruppe jeweils entweder über eine Bindung mit dem dreizähnigen Liganden verknüpft oder mit diesem über zwei C-C-Bindungen anelliert ist, -COR, -COOR, -COOH, OH, OR, NRR', wobei R und R' unabhängig voneinander Wasserstoff oder eine Aryl- oder Alkylgruppe mit 1 bis 20 C-Atomen bedeuten,
steht und
R² den aromatischen Ring ein- oder mehrfach substituieren kann;
L für einen neutralen Liganden steht;
m = 0, 1, 2 oder 3 ist;
Z⁻ für einen monoanionischen Liganden ausgewählt aus der Gruppe bestehend aus: Sulfonaten, Carboxylaten, Pseudohalogeniden, [BF₄]⁻ [PF₆]⁻, [SbF₆]⁻, Nitrat, Hydrogensulfat, Fluorosulfat und Methylsulfat steht;
und
das Verfahren unter halogenfreien Reaktionsbedingungen durchgeführt wird.

2. Verfahren nach Anspruch 1, bei dem L ausgewählt ist aus der Gruppe bestehend aus:
Wasser, Azaaromaten, cyclische oder acyclische Amine, cyclische oder acyclische Carbonsäureamide, cyclische oder acyclische Carbonsäureester, cyclische oder acyclische Ketone, cyclische oder acyclische Ketimine, aliphatische Ether, aliphatische oder aromatische Alkohole, Sulfoxide, Formamide, cyclische oder acyclische Organocarbonate, Nitrile.

3. Verfahren nach Anspruch 1 oder 2, bei dem Z⁻ ausgewählt ist aus der Gruppe bestehend aus:
Tosylat, Mesylat, Acetat, Benzoat, Cyanid, Cyanat, Thiocyanat, Azid, Nitrat, Hydrogensulfat und Methylsulfat.

4. Verfahren nach Anspruch 1, bei dem sich der dianionische Chelatligand (O~N~O)²⁻ durch Deprotonierung aller OH-Funktionen von neutralen Chelatliganden ausgewählt aus der Gruppe bestehend aus: 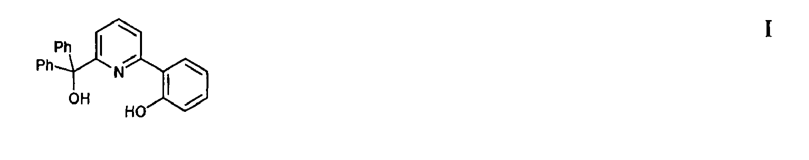 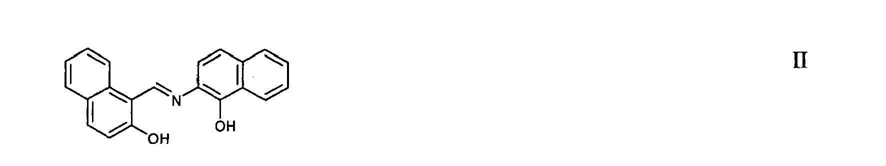 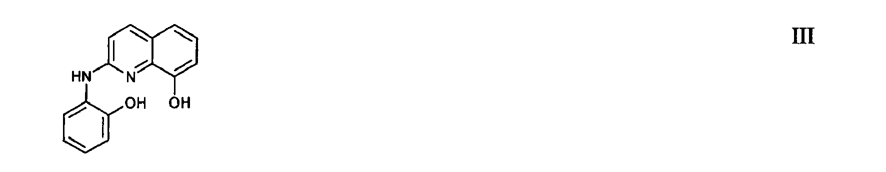 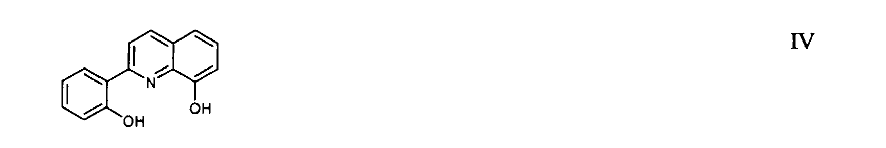 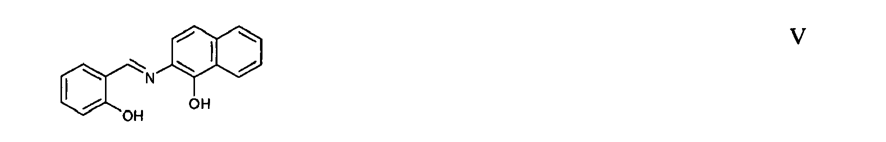 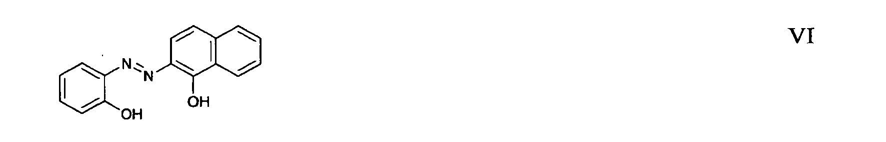 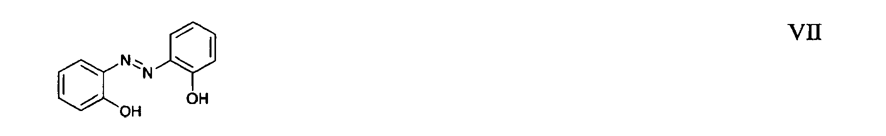 ableitet.

5. Verfahren nach einem der Ansprüche 1 bis 4, bei dem der Katalysator in einer Konzentration von 0,01 Mol-% bis 10 Mol%, bezogen auf ein Mol primärerer Aminogruppen, vorliegt.

6. Verfahren nach einem der Ansprüche 1 bis 5, bei dem die oxidative Carbonylierung bei Temperaturen von 20 °C bis 260 °C und bei einem absoluten Kohlenmonoxidpartialdruck von 1,0 bar bis 150 bar sowie bei einem absoluten Sauerstoffpartialdruck von 0,10 bar bis 20 bar durchgeführt wird.

7. Verfahren nach einem der Ansprüche 1 bis 6, bei dem die oxidative Carbonylierung der organischen Amine in Gegenwart von organischen, Hydroxylgruppen tragenden Verbindungen durchgeführt wird und zur Herstellung von Urethanen oder Gemischen aus Urethanen und Harnstoffen führt.

8. Verfahren nach Anspruch 7, bei dem die organischen, Hydroxylgruppen tragenden Verbindungen ausgewählt sind aus der Gruppe bestehend aus:
Methanol, Ethanol, n-Butanol, iso-Butanol, sek-Butanol, n-Propanol, iso-Propanol.

9. Verfahren nach einem der Ansprüche 1 bis 8, bei dem als organische Amine aliphatische, cycloaliphatische, araliphatische und / oder aromatische Mono- oder Diamine eingesetzt werden.

10. Verfahren nach Anspruch 9, bei dem Anilin oder Toluylendiamin als aromatische Amine eingesetzt werden.

11. Verwendung von Übergangsmetallkomplexen enthaltend das Strukturmerkmal
[Mⁿ⁺(O~N~O)²⁻]⁽ⁿ⁻²⁾⁺(L)ₘ(Z-)ₙ₋₂, worin
M Co bezeichnet;
n = 2 oder 3 ist;
(O~N~O)²⁻ für einen dreizähnigen, dianionischen, mit M über zwei Sauerstoffatome und ein Stickstoffatom koordinierenden Chelatliganden steht, welcher sich durch Deprotonierung aller OH-Funktionen, welche nicht Bestandteil der Substituenten R¹ oder R² sind, von neutralen Chelatliganden ausgewählt aus der Gruppe bestehend aus: 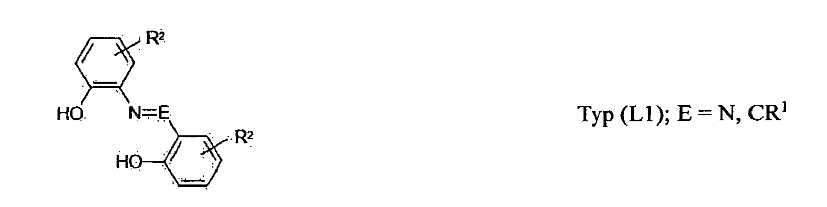 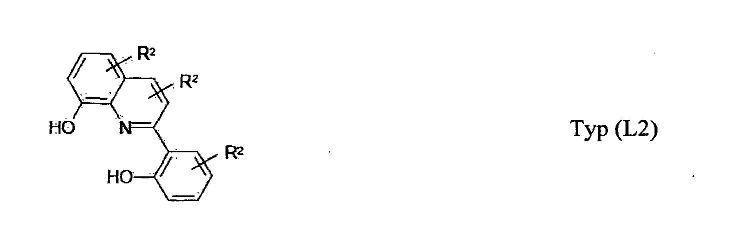 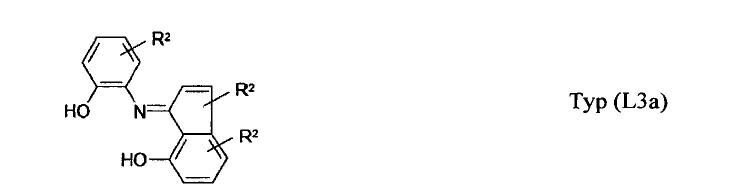 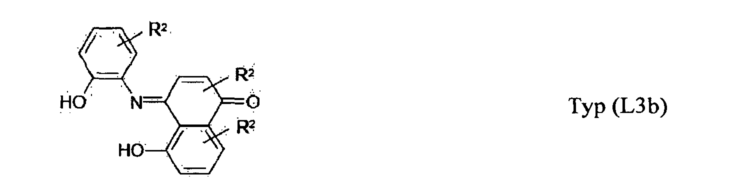 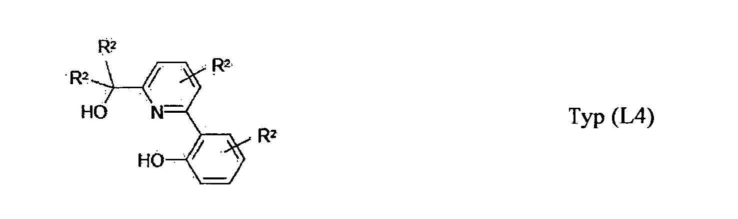 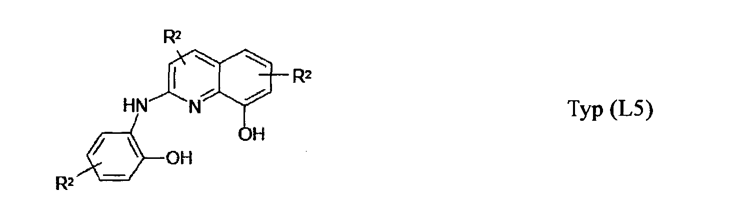 ableitet und worin
R¹ für einen Substituenten ausgewählt aus der Gruppe bestehend aus:
Wasserstoff, eine Alkylgruppe mit 1 bis 20 Kohlenstoffatomen, eine Aryl- oder Heteroarylgruppe, eine OR-Gruppe, in der R für Wasserstoff oder eine Alkylgruppe mit 1 bis 20 Kohlenstoffatomen steht, eine NRR'-Gruppe, in der R und R' unabhängig voneinander Wasserstoff oder eine Aryl- oder Alkylgruppe mit 1 bis 20 C-Atomen bedeuten,
steht;
R² für einen Substituenten ausgewählt der Gruppe bestehend aus:
Wasserstoff, eine Alkylgruppe mit 1 bis 20 Kohlenstoffatomen, eine Arylgruppe oder Heteroarylgruppe, wobei die Arylgruppe oder Heteroarylgruppe jeweils entweder über eine Bindung mit dem dreizähnigen Liganden verknüpft oder mit diesem über zwei C-C-Bindungen anelliert ist, -COR, -COOR, -COOH, OH, OR, NRR', wobei R und R' unabhängig voneinander Wasserstoff oder eine Aryl- oder Alkylgruppe mit 1 bis 20 C-Atomen bedeuten,
steht und
R² den aromatischen Ring ein- oder mehrfach substituieren kann;
L für einen neutralen Liganden ausgewählt aus der Gruppe bestehend aus:
Wasser, Azaaromaten, cyclische oder acyclische Amine, cyclische oder acyclische Carbonsäureamide, cyclische oder acyclische Carbonsäureester, cyclische oder acyclische Ketone, cyclische oder acyclische Ketimine, aliphatische Ether, aliphatische oder aromatische Alkohole, Sulfoxide, cyclische oder acyclische Organocarbonate, Nitrile
steht;
m = 0, 1, 2 oder 3 ist
und
Z⁻ für einen monoanionischen Liganden ausgewählt aus der Gruppe bestehend aus:
Sulfonaten, Carboxylaten, Pseudohalogeniden, [BF₄]⁻ [PF₆]⁻, [SbF₆]⁻, Nitrat, Hydrogensulfat, Fluorosulfat und Methylsulfat,
steht
als Katalysatoren in Verfahren zur Herstellung von Urethanen oder Harnstoffen oder Gemischen aus Urethanen und Harnstoffen.

12. Verwendung von Übergangsmetallkomplexen enthaltend das Strukturmerkmal
[Mⁿ⁺(O~N~O)²⁻]⁽ⁿ⁻²⁾⁺(L)ₘ(Z⁻)ₙ₋₂, worin
M Co bezeichnet;
n = 2 oder 3 ist;
(O∼N∼O)²⁻ für einen dreizähnigen, dianionischen, mit M über zwei Sauerstoffatome und ein Stickstoffatom koordinierenden Chelatliganden steht, welcher sich durch Deprotonierung aller OH-Funktionen, welche nicht Bestandteil der Substituenten R¹ oder R² sind, von neutralen Chelatliganden ausgewählt aus der Gruppe bestehend aus: 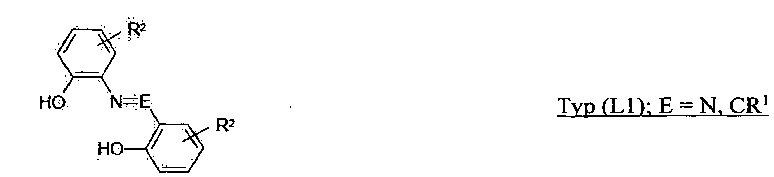 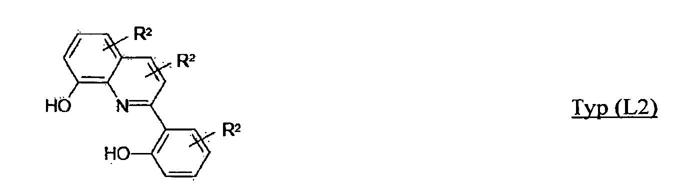 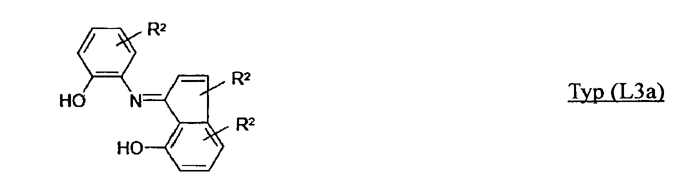 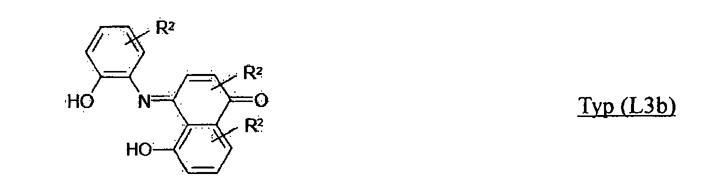 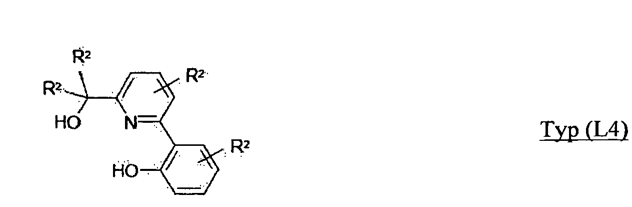 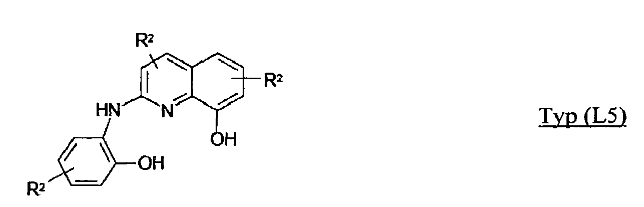 ableitet und worin
R¹ für einen Substituenten ausgewählt aus der Gruppe bestehend aus:
Wasserstoff, eine Alkylgruppe mit 1 bis 20 Kohlenstoffatomen, eine Aryl- oder Heteroarylgruppe, eine OR-Gruppe, in der R für Wasserstoff oder eine Alkylgruppe mit 1 bis 20 Kohlenstoffatomen steht, eine NRR'-Gruppe, in der R und R' unabhängig voneinander Wasserstoff oder eine Aryl- oder Alkylgruppe mit 1 bis 20 C-Atomen bedeuten,
steht;
R² für einen Substituenten ausgewählt der Gruppe bestehend aus:
Wasserstoff, eine Alkylgruppe mit 1 bis 20 Kohlenstoffatomen, eine Arylgruppe oder Heteroarylgruppe, wobei die Arylgruppe oder Heteroarylgruppe jeweils entweder über eine Bindung mit dem dreizähnigen Liganden verknüpft oder mit diesem über zwei C-C-Bildungen anelliert ist, -COR, -COOR, -COOH, OH, OR, NRR', wobei R und R' unabhängig voneinander Wasserstoff oder eine Aryl- oder Alkylgruppe mit 1 bis 20 C-Atomen bedeuten,
steht und
R² den aromatischen Ring ein- oder mehrfach substituieren kann;
L für einen neutralen Liganden ausgewählt aus der Gruppe bestehend aus:
Wasser Azaaromaten, cyclische oder acyclische Amine, cyclische oder acyclische Carbonsäureamide, cyclische oder acyclische Carbonsäureester, cyclische oder acyclische Ketone, cyclische oder acyclische Ketimine, aliphatische Ether, aliphatische oder aromatische Alkohole, Sulfoxide, cyclische oder acyclische Organocarbonate, Nitrile
steht;
m = 0, 1, 2 oder 3 ist
und
Z⁻ für einen monoanionischen Liganden ausgewählt aus der Gruppe bestehend aus:
Sulfonaten Carboxylaten Pseudohalogeniden, [BF₄]⁻ FPF₆]⁻, [SbF₆]⁻, Nitrat, Hydrogensulfat, Fluorosulfat und Methylsulfat,
steht
in der Herstellung von Isocyanaten.

13. Verwendung nach Anspruch 11 oder 12, worin
(O∼N-O)²⁻ für einen dreizähnigen, dianionischen, mit M über zwei Sauerstoffatome und ein Stickstoffatom koordinierenden Chelatliganden steht, welcher sich durch Deprotonierung aller OH-Funktionen von neutralen Chelatliganden ausgewählt aus der Gruppe bestehend aus: 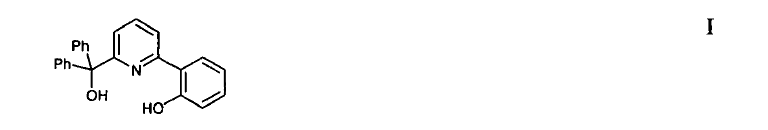 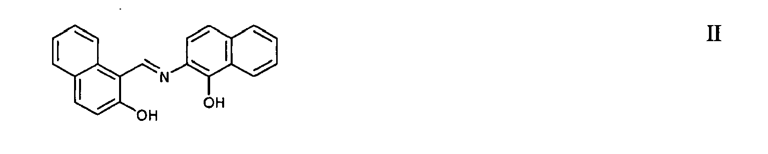 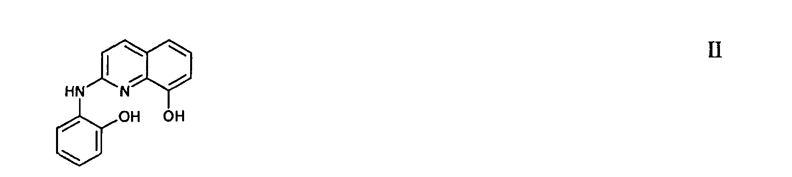 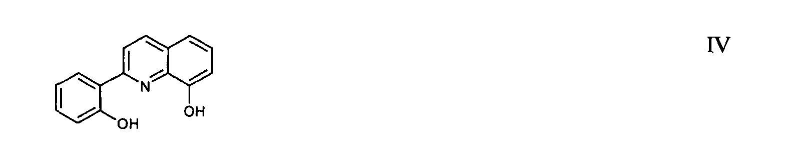 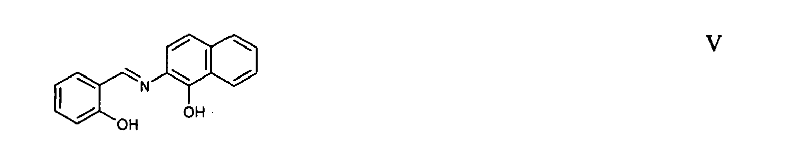 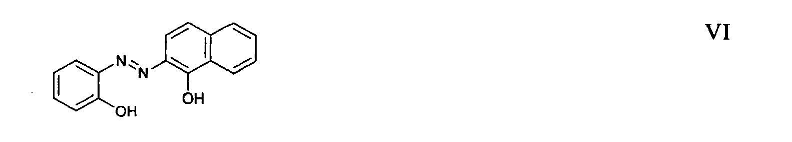 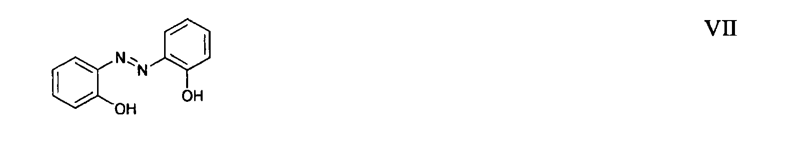 ableitet.

14. Verwendung nach Anspruch 11, 12 oder 13, bei der die dreizähnigen, dianionischen, Liganden (O∼N∼O)²⁻ drei meridionale Koordinationsstellen des Cobalts besetzen.

## Claims

1. Process for the preparation of urethanes or ureas or mixtures of urethanes and ureas by the oxidative carbonylation of organic amines containing primary amino groups in the presence of carbon monoxide, oxygen and a catalyst, **characterized in that**
the catalyst used is a transition metal complex containing the structural feature [Mⁿ⁺(O~N~O)²⁻]ⁿ⁻²⁺(L)ₘ(Z⁻)ₙ₋₂,
M being Co,
n being 2 or 3;
(O~N~O)²⁻ being a tridentate, dianionic chelating ligand coordinating with M via two oxygen atoms and one nitrogen atom, which is derived by deprotonation of all the OH groups (that are not part of the substituents R¹ or R²) of neutral chelating ligands selected from the group comprising: 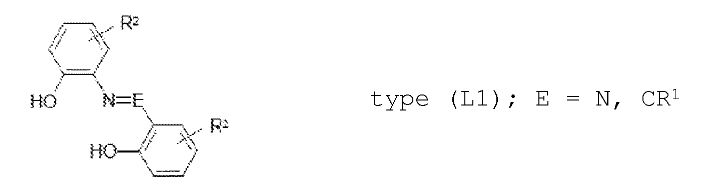 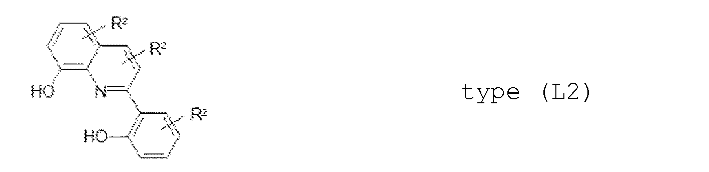 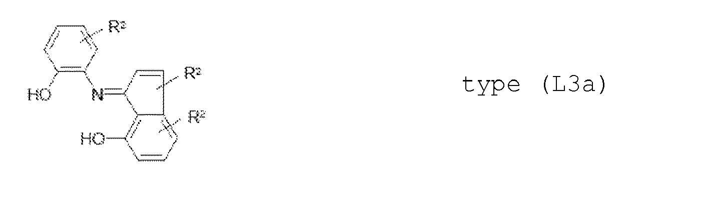 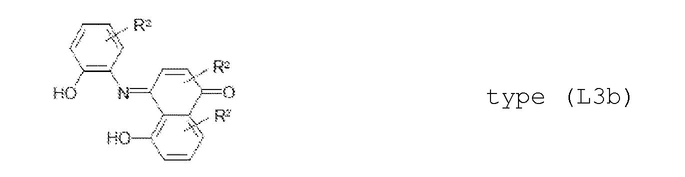 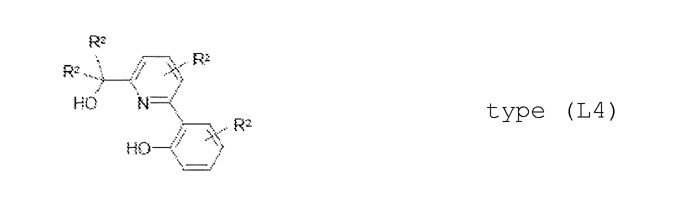 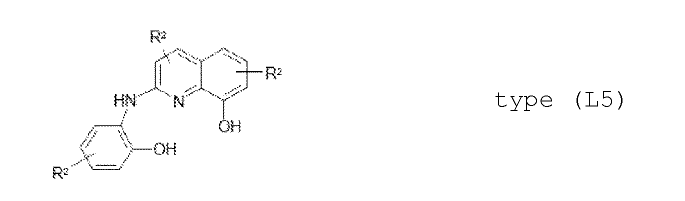
R¹ being a substituent selected from the group comprising:
hydrogen, an alkyl group having 1 to 20 carbon atoms, an aryl or heteroaryl group, a group OR, in which R is hydrogen or an alkyl group having 1 to 20 carbon atoms, and a group NRR', in which R and R' independently of one another are hydrogen or an aryl or alkyl group having 1 to 20 C atoms;
R² being a substituent selected from the group comprising:
hydrogen, an alkyl group having 1 to 20 carbon atoms, an aryl group or heteroaryl group, the aryl group or heteroaryl group each being either joined via a bond to the tridentate ligand or fused to the latter via two C-C bonds, -COR, - COOR, -COOH, OH, OR and NRR', in which R and R' independently of one another are hydrogen or an aryl or alkyl group having 1 to 20 C atoms,
and
it being possible for R² to substitute the aromatic ring one or more times;
L being a neutral ligand;
m being 0, 1, 2 or 3;
Z⁻ being a monoanionic ligand selected from the group comprising:
sulfonates, carboxylates, pseudohalides, [BF₄]⁻, [PF₆]⁻, [SbF₆]⁻, nitrate, hydrogensulfate, fluorosulfate and methylsulfate;
and
the process is carried out under halogen-free reaction conditions.

2. Process according to Claim 1 wherein L is selected from the group comprising:
water, azaaromatics, cyclic or acyclic amines, cyclic or acyclic carboxamides, cyclic or acyclic carboxylic acid esters, cyclic or acyclic ketones, cyclic or acyclic ketimines, aliphatic ethers, aliphatic or aromatic alcohols, sulfoxides, formamides, cyclic or acyclic organocarbonates and nitriles.

3. Process according to Claim 1 or 2 wherein Z⁻ is selected from the group comprising:
tosylate, mesylate, acetate, benzoate, cyanide, cyanate, thiocyanate, azide, nitrate, hydrogensulfate and methylsulfate.

4. Process according to Claim 1 wherein the dianionic chelating ligand (O~N~O)²⁻ is derived by deprotonation of all the OH groups of neutral chelating ligands selected from the group comprising:

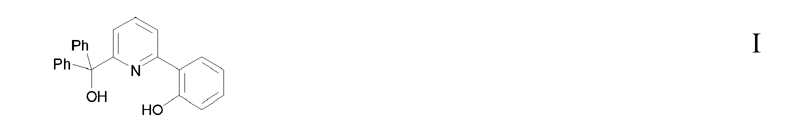

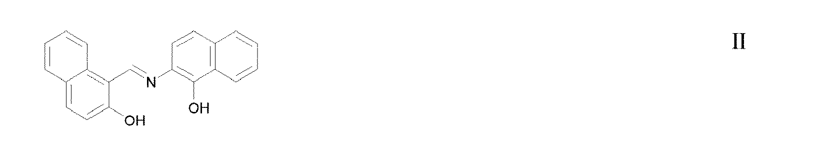

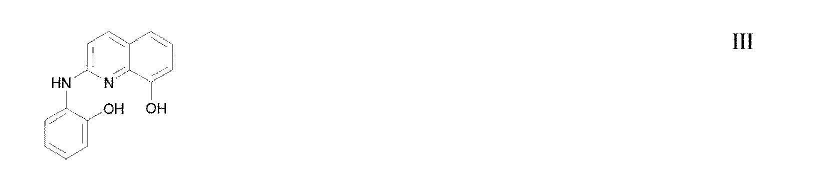

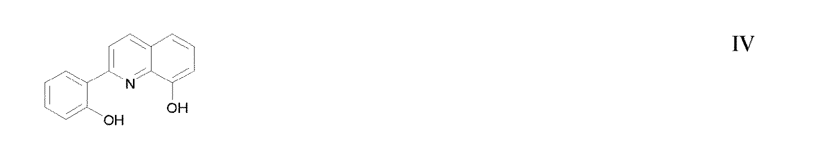

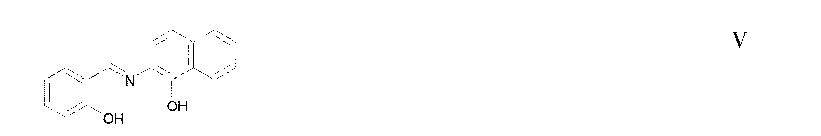

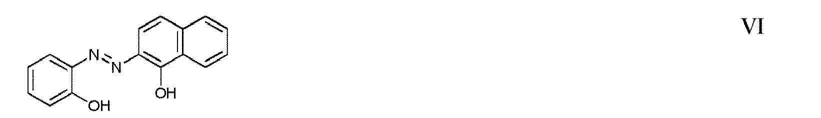

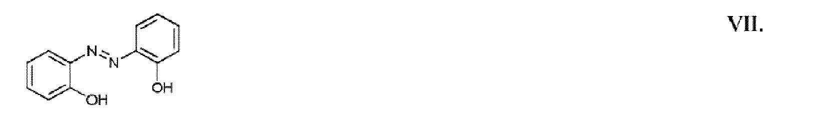

5. Process according to one of Claims 1 to 4 wherein the catalyst is present in a concentration of 0.01 mol% to 10 mol%, based on one mol of primary amino groups.

6. Process according to one of Claims 1 to 5 wherein the oxidative carbonylation is carried out at temperatures of 20°C to 260°C, at an absolute carbon monoxide partial pressure of 1.0 bar to 150 bar and at an absolute oxygen partial pressure of 0.10 bar to 20 bar.

7. Process according to one of Claims 1 to 6 wherein the oxidative carbonylation of organic amines is carried out in the presence of organic compounds carrying hydroxyl groups and leads to the preparation of urethanes or mixtures of urethanes and ureas.

8. Process according to Claim 7 wherein the organic compounds carrying hydroxyl groups are selected from the group comprising:
methanol, ethanol, n-butanol, isobutanol, sec-butanol, n-propanol and isopropanol.

9. Process according to one of Claims 1 to 8 wherein the organic amines used are aliphatic, cycloaliphatic, araliphatic and/or aromatic mono- or diamines.

10. Process according to Claim 9 wherein the aromatic amines used are aniline or toluylenediamine.

11. Use of transition metal complexes containing the structural feature
[Mⁿ⁺(O~N~O)²⁻]⁽ⁿ⁻²⁾⁺(L)ₘ(Z⁻)ₙ₋₂,
M being Co;
n being 2 or 3;
(O~N~O)²⁻ being a tridentate, dianionic chelating ligand coordinating with M via two oxygen atoms and one nitrogen atom, which is derived by deprotonation of all the OH groups (that are not part of the substituents R¹ or R²) of neutral chelating ligands selected from the group comprising: 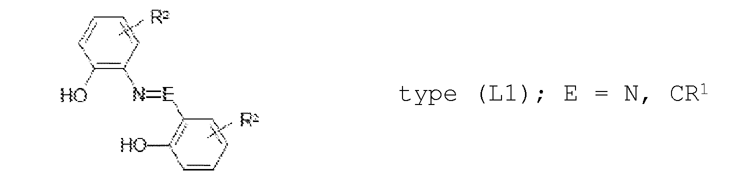 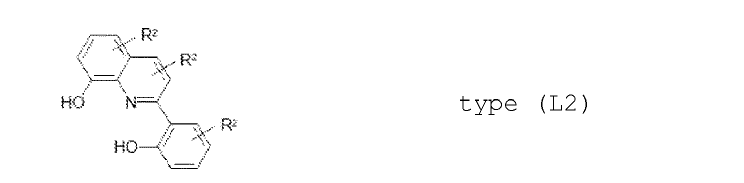 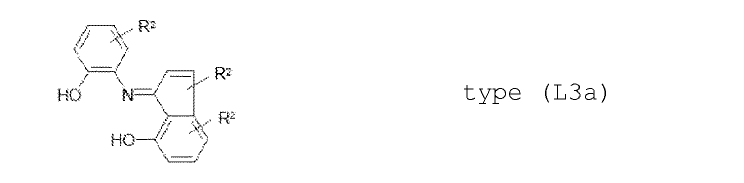 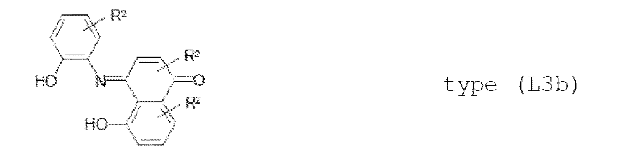 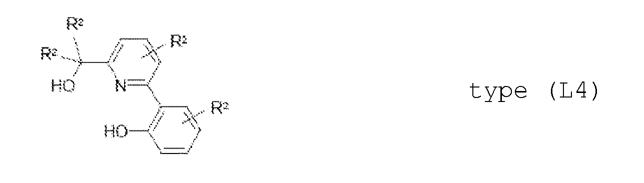 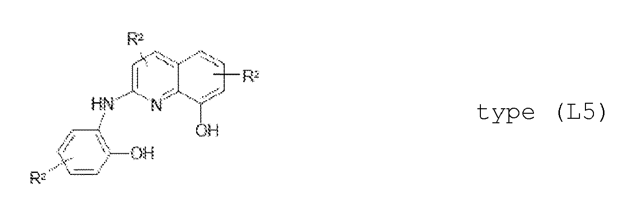
R¹ being a substituent selected from the group comprising:
hydrogen, an alkyl group having 1 to 20 carbon atoms, an aryl or heteroaryl group, a group OR, in which R is hydrogen or an alkyl group having 1 to 20 carbon atoms, and a group NRR', in which R and R' independently of one another are hydrogen or an aryl or alkyl group having 1 to 20 C atoms;
R² being a substituent selected from the group comprising:
hydrogen, an alkyl group having 1 to 20 carbon atoms, an aryl group or heteroaryl group, the aryl group or heteroaryl group each being either joined via a bond to the tridentate ligand or fused to the latter via two C-C bonds, -COR, - COOR, -COOH, OH, OR and NRR', in which R and R' independently of one another are hydrogen or an aryl or alkyl group having 1 to 20 C atoms,
and
it being possible for R² to substitute the aromatic ring one or more times;
L being a neutral ligand selected from the group comprising: water, azaaromatics, cyclic or acyclic amines, cyclic or acyclic carboxamides, cyclic or acyclic carboxylic acid esters, cyclic or acyclic ketones, cyclic or acyclic ketimines, aliphatic ethers, aliphatic or aromatic alcohols, sulfoxides, cyclic or acyclic organocarbonates and nitriles;
m being 0, 1, 2 or 3
and
Z⁻ being a monoanionic ligand selected from the group comprising:
sulfonates, carboxylates, pseudohalides, [BF₄]⁻, [PF₆]⁻, [SbF₆]⁻, nitrate, hydrogensulfate, fluorosulfate and methylsulphate,
as catalysts in processes for the preparation of urethanes or ureas or mixtures of urethanes and ureas.

12. Use of transition metal complexes containing the structural feature
[Mⁿ⁺(O~N~O)²⁻]⁽ⁿ⁻²⁾⁺(L)ₘ(Z⁻)ₙ₋₂,
M being Co;
n being 2 or 3;
(O~N~O)²⁻ being a tridentate, dianionic chelating ligand coordinating with M via two oxygen atoms and one nitrogen atom, which is derived by deprotonation of all the OH groups (that are not part of the substituents R¹ or R²) of neutral chelating ligands selected from the group comprising: 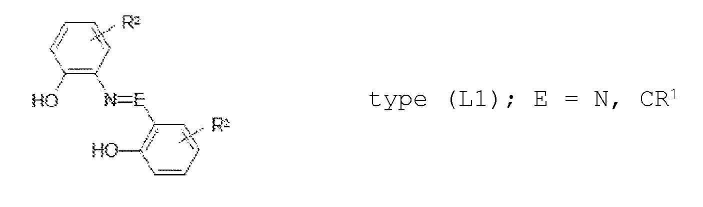 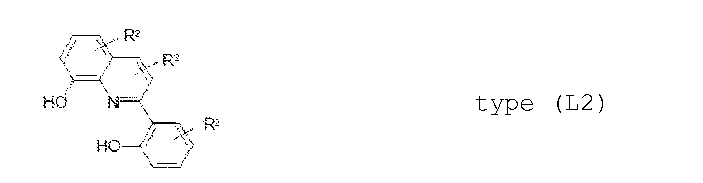 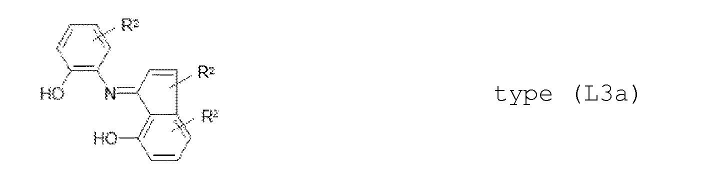 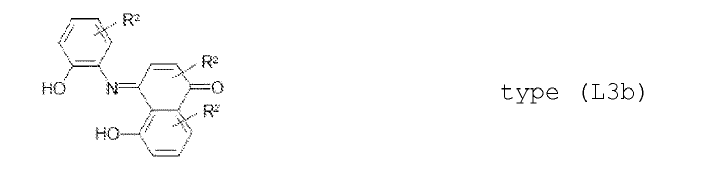 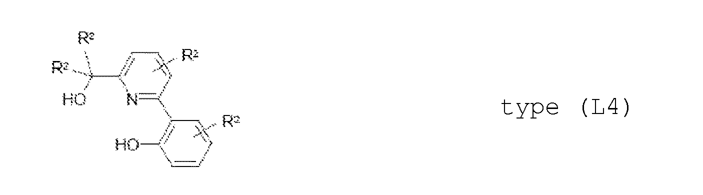 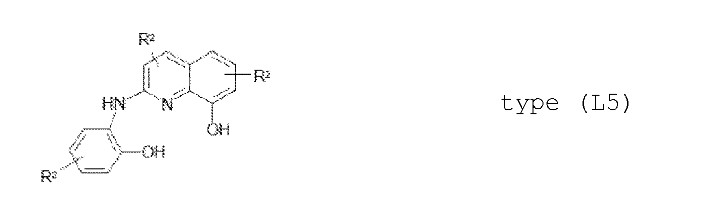
R¹ being a substituent selected from the group comprising:
hydrogen, an alkyl group having 1 to 20 carbon atoms, an aryl or heteroaryl group, a group OR, in which R is hydrogen or an alkyl group having 1 to 20 carbon atoms, and a group NRR', in which R and R' independently of one another are hydrogen or an aryl or alkyl group having 1 to 20 C atoms;
R² being a substituent selected from the group comprising:
hydrogen, an alkyl group having 1 to 20 carbon atoms, an aryl group or heteroaryl group, the aryl group or heteroaryl group each being either joined via a bond to the tridentate ligand or fused to the latter via two C-C bonds, -COR, - COOR, -COOH, OH, OR and NRR', in which R and R' independently of one another are hydrogen or an aryl or alkyl group having 1 to 20 C atoms,
and
it being possible for R² to substitute the aromatic ring one or more times;
L being a neutral ligand selected from the group comprising:
water, azaaromatics, cyclic or acyclic amines, cyclic or acyclic carboxamides, cyclic or acyclic carboxylic acid esters, cyclic or acyclic ketones, cyclic or acyclic ketimines, aliphatic ethers, aliphatic or aromatic alcohols, sulfoxides, cyclic or acyclic organocarbonates and nitriles;
m being 0, 1, 2 or 3
and
Z⁻ being a monoanionic ligand selected from the group comprising:
sulfonates, carboxylates, pseudohalides, [BF₄]⁻, [PF₆]⁻, [SbF₆]⁻, nitrate, hydrogensulfate, fluorosulfate and methylsulphate,
in the preparation of isocyanates.

13. Use according to Claim 11 or 12 wherein
(O~N~O)²⁻ is a tridentate, dianionic chelating ligand coordinating with M via two oxygen atoms and one nitrogen atom, which is derived by deprotonation of all the OH groups of neutral chelating ligands selected from the group comprising:

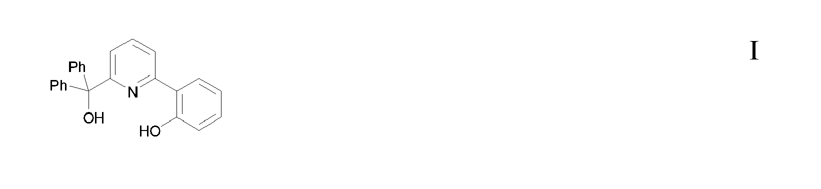

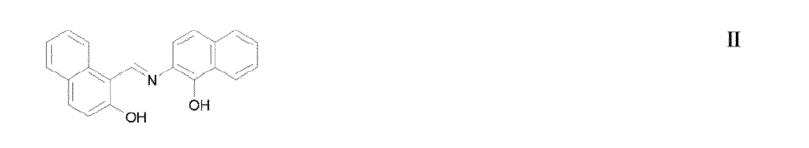

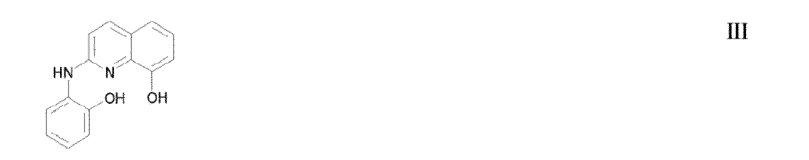

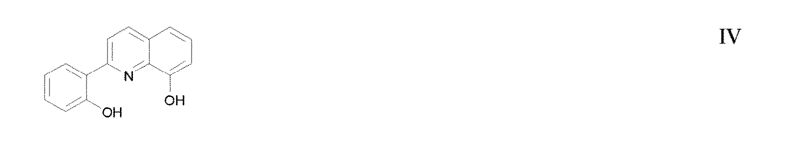

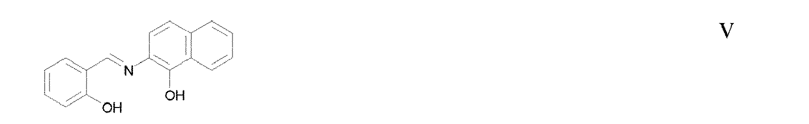

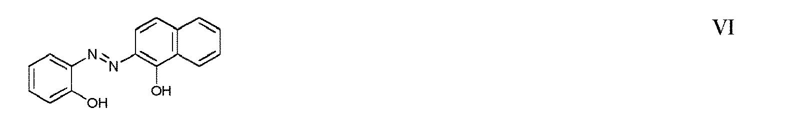

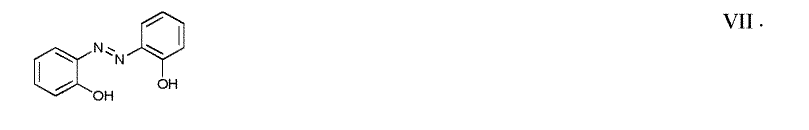

14. Use according to Claim 11, 12 or 13 wherein the tridentate, dianionic ligands (O~N~O)²⁻ occupy three meridional coordination sites around the cobalt.

## Revendications

1. Procédé pour la préparation d'uréthanes ou d'urées ou de mélanges d'uréthanes et d'urées par carbonylation oxydante d'amines organiques contenant des groupes amino primaires en présence de monoxyde de carbone, d'oxygène et d'un catalyseur, **caractérisé en ce**
**qu'**on utilise comme catalyseur un complexe de métal de transition, comportant la caractéristique de structure [Mⁿ⁺(O~N~O)²⁻]⁽ⁿ⁻²⁾⁺(L)ₘ(Z⁻)ₙ₋₂, dans laquelle
M représente Co,
n est égal à 2 ou 3 ;
(O~N~O)²⁻ représente un ligand chélateur tridenté, dianionique, coordinant M avec deux atomes d'oxygène et un atome d'azote, qui dérive par déprotonation de toutes les fonctions OH, qui ne font pas partie des substituants R¹ ou R², de ligands chélateurs neutres choisis dans le groupe constitué par : 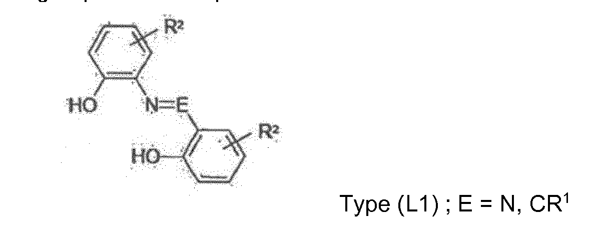 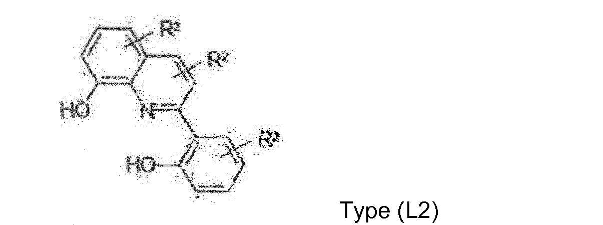 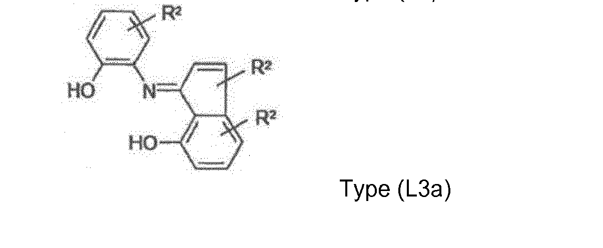 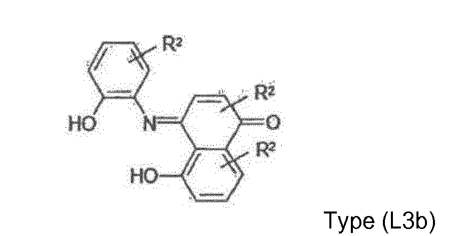 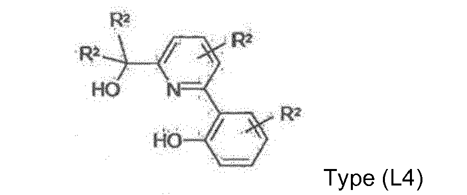 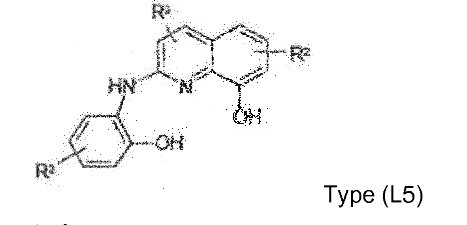 et où
R¹ représente un substituant choisi dans le groupe constitué par :
un atome d'hydrogène, un groupe alkyle ayant de 1 à 20 atomes de carbone, un groupe aryle ou hétéroaryle, un groupe OR, dans lequel R représente un atome d'hydrogène ou un groupe alkyle ayant de 1 à 20 atomes de carbone, un groupe NRR', dans lequel R et R' représentent indépendamment l'un de l'autre un atome d'hydrogène ou un groupe aryle ou alkyle ayant de 1 à 20 atomes de carbone ;
R² représente un substituant choisi dans le groupe constitué par :
un atome d'hydrogène, un groupe alkyle ayant de 1 à 20 atomes de carbone, un groupe aryle ou un groupe hétéroaryle, le groupe aryle ou le groupe hétéroaryle étant respectivement soit lié par une liaison au ligand tridenté soit soudé à ce dernier par deux liaisons C-C, un groupe -COR, -COOR, -COOH, OH, OR, NRR', R et R' représentant indépendamment l'un de l'autre un atome d'hydrogène ou un groupe aryle ou alkyle ayant de 1 à 20 atomes de carbone,
et
R² peut substituer une ou plusieurs fois le cycle aromatique ;
L représente un ligand neutre ;
m est égal à 0, 1, 2 ou 3 ;
Z⁻ représente un ligand monoanionique choisi dans le groupe constitué par :
des sulfonates, carboxylates, pseudohalogénures, [BF₄]⁻, [PF₆]⁻, [SbF₆]⁻, le nitrate, l'hydrogénosulfate, le fluorosulfate et le méthylsulfate ;
et
on effectue le procédé dans des conditions réactionnelles sans halogène.

2. Procédé selon la revendication 1, dans lequel L est choisi dans le groupe constitué par :
l'eau, des composés aza-aromatiques, des amines cycliques ou acycliques, des carboxamides cycliques ou acycliques, des esters d'acides carboxyliques cycliques ou acycliques, des cétones cycliques ou acycliques, des cétimines cycliques ou acycliques, des éthers aliphatiques, des alcools aliphatiques ou aromatiques, des sulfoxydes, des formamides, des organocarbonates cycliques ou acycliques, des nitriles.

3. Procédé selon la revendication 1 ou 2, dans lequel Z⁻ est choisi dans le groupe constitué par :
le tosylate, le mésylate, l'acétate, le benzoate, le cyanure, le cyanate, le thiocyanate, l'azoture, le nitrate, l'hydrogénosulfate et le méthylsulfate.

4. Procédé selon la revendication 1, dans lequel le ligand chélateur dianionique (O∼N∼O)²⁻ dérive par déprotonation de toutes les fonctions OH de ligands chélateurs neutres choisis dans le groupe constitué par :

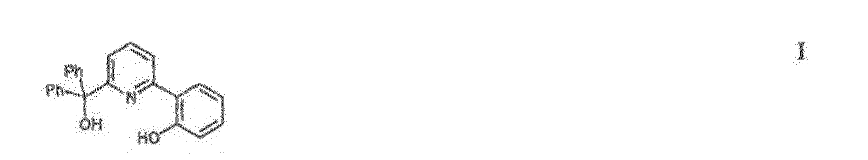

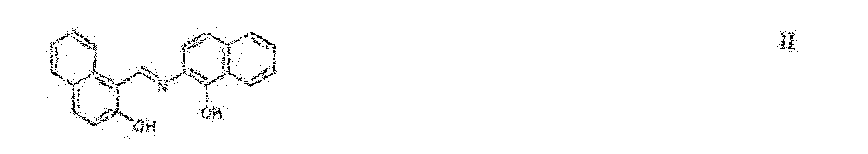

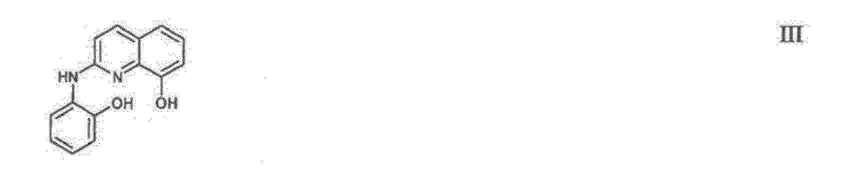

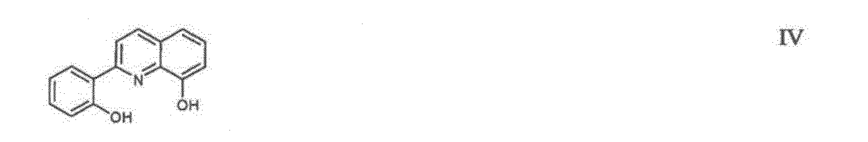

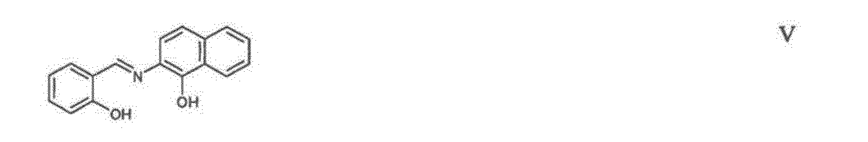

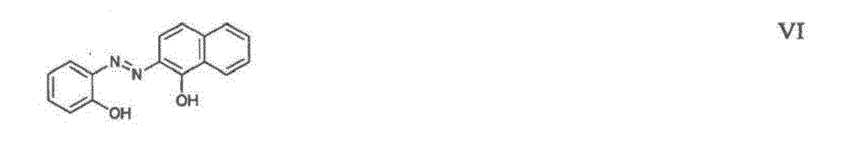

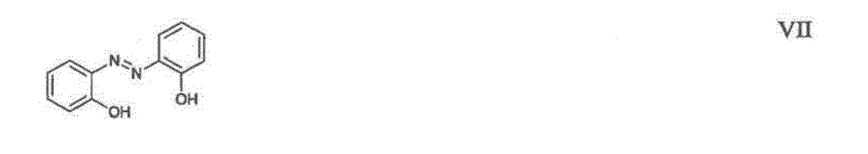

5. Procédé selon l'une quelconque des revendications 1 à 4, dans lequel le catalyseur est présent à une concentration de 0,01 % en moles à 10 % en moles, par rapport à une mole de groupes amino primaires.

6. Procédé selon l'une quelconque des revendications 1 à 5, dans lequel on effectue la carbonylation oxydante à des températures de 20 °C à 260 °C et sous une pression partielle absolue de monoxyde de carbone de 1,0 bar à 150 bars ainsi que sous une pression partielle absolue d'oxygène de 0,10 bar à 20 bars.

7. Procédé selon l'une quelconque des revendications 1 à 6, dans lequel la carbonylation oxydante des amines organiques est effectuée en présence de composés organiques portant des groupes hydroxy et conduit à la production d'uréthanes ou de mélanges d'uréthanes et d'urées.

8. Procédé selon la revendication 7, dans lequel les composés organiques portant des groupes hydroxy sont choisis dans le groupe constitué par :
le méthanol, l'éthanol, le n-butanol, l'isobutanol, le sec-butanol, le n-propanol, l'isopropanol.

9. Procédé selon l'une quelconque des revendications 1 à 8, dans lequel on utilise comme amines organiques des mono- ou diamines aliphatiques" cycloaliphatiques, araliphatiques et/ou aromatiques.

10. Procédé selon la revendication 9, dans lequel on utilise comme amines aromatiques l'aniline ou la toluylènediamine.

11. Utilisation de complexes de métaux de transition, comportant la caractéristique de structure [Mⁿ⁺(O~N~O)²⁻]⁽ⁿ⁻²⁾⁺(L)ₘ(Z⁻)ₙ₋₂, dans laquelle
M représente Co,
n est égal à 2 ou 3 ;
(O~N~O)²⁻ représente un ligand chélateur tridenté, dianionique, coordinant M avec deux atomes d'oxygène et un atome d'azote, qui dérive par déprotonation de toutes les fonctions OH, qui ne font pas partie des substituants R¹ ou R², de ligands chélateurs neutres choisis dans le groupe constitué par : 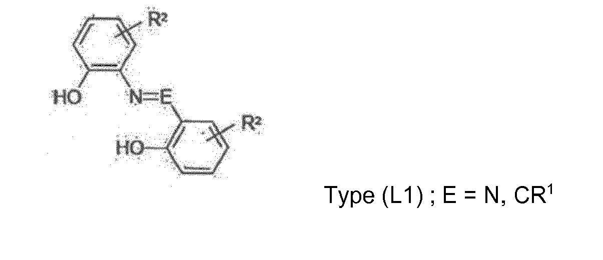 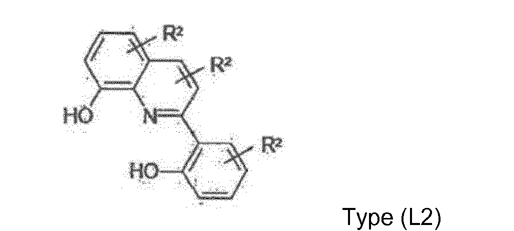 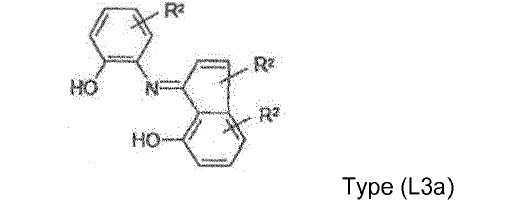 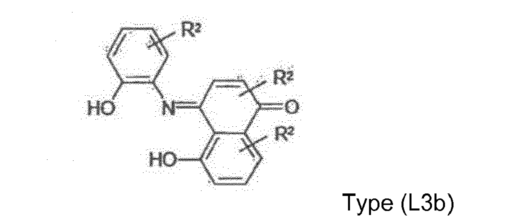 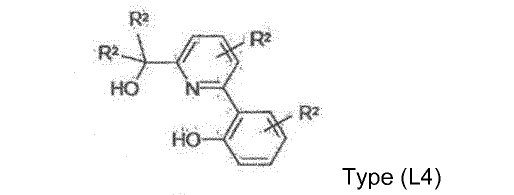 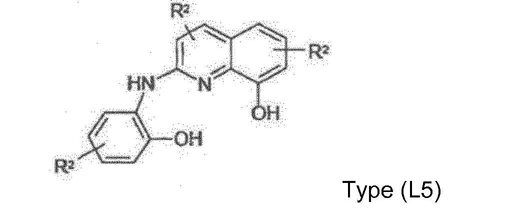 et où
R¹ représente un substituant choisi dans le groupe constitué par :
un atome d'hydrogène, un groupe alkyle ayant de 1 à 20 atomes de carbone, un groupe aryle ou hétéroaryle, un groupe OR, dans lequel R représente un atome d'hydrogène ou un groupe alkyle ayant de 1 à 20 atomes de carbone, un groupe NRR', dans lequel R et R' représentent indépendamment l'un de l'autre un atome d'hydrogène ou un groupe aryle ou alkyle ayant de 1 à 20 atomes de carbone ;
R² représente un substituant choisi dans le groupe constitué par :
un atome d'hydrogène, un groupe alkyle ayant de 1 à 20 atomes de carbone, un groupe aryle ou un groupe hétéroaryle, le groupe aryle ou le groupe hétéroaryle étant respectivement soit lié par une liaison au ligand tridenté soit soudé à ce dernier par deux liaisons C-C, un groupe -COR, -COOR, -COOH, OH, OR, NRR', R et R' représentant indépendamment l'un de l'autre un atome d'hydrogène ou un groupe aryle ou alkyle ayant de 1 à 20 atomes de carbone,
et
R² peut substituer une ou plusieurs fois le cycle aromatique ;
L représente un ligand neutre choisi dans le groupe constitué par :
l'eau, des composés aza-aromatiques, des amines cycliques ou acycliques, des carboxamides cycliques ou acycliques, des esters d'acides carboxyliques cycliques ou acycliques, des cétones cycliques ou acycliques, des cétimines cycliques ou acycliques, des éthers aliphatiques, des alcools aliphatiques ou aromatiques, des sulfoxydes, des organocarbonates cycliques ou acycliques, des nitriles ;
m est égal à 0, 1, 2 ou 3
et
Z⁻ représente un ligand monoanionique choisi dans le groupe constitué par:
des sulfonates, carboxylates, pseudohalogénures, [BF₄]⁻, [PF₆]⁻, [SbF₆]⁻, le nitrate, l'hydrogénosulfate, le fluorosulfate et le méthylsulfate,
en tant que catalyseurs dans des procédés pour la préparation d'uréthanes ou d'urée ou de mélanges d'uréthanes et d'urées.

12. Utilisation de complexes de métaux de transition, comportant la caractéristique de structure [Mⁿ⁺(O~N~O)²⁻]⁽ⁿ⁻²⁾⁺(L)ₘ(Z⁻)ₙ₋₂, dans laquelle
M représente Co,
n est égal à 2 ou 3 ;
(O~N~O)²⁻ représente un ligand chélateur tridenté, dianionique, coordinant M avec deux atomes d'oxygène et un atome d'azote, qui dérive par déprotonation de toutes les fonctions OH, qui ne font pas partie des substituants R¹ ou R², de ligands chélateurs neutres choisis dans le groupe constitué par : 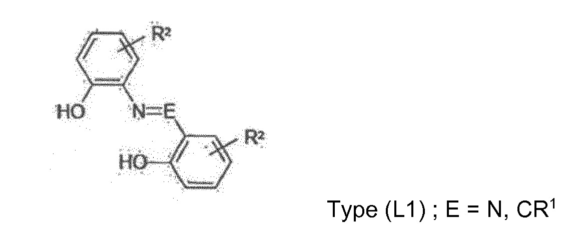 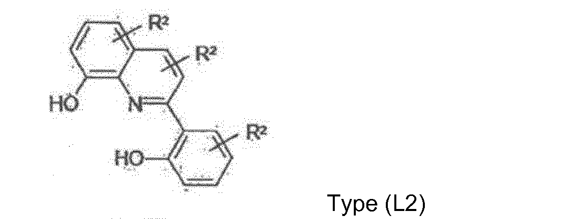 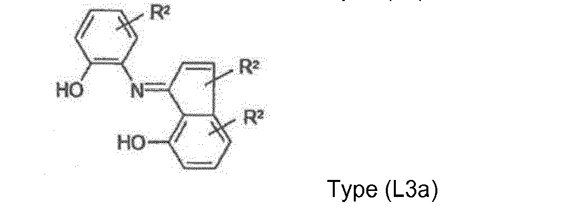 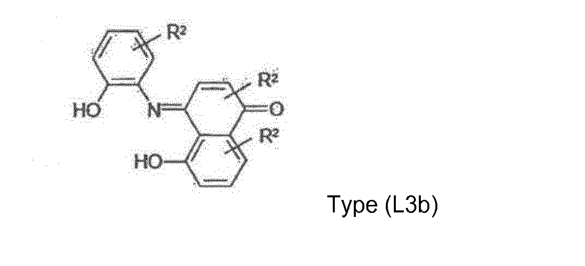 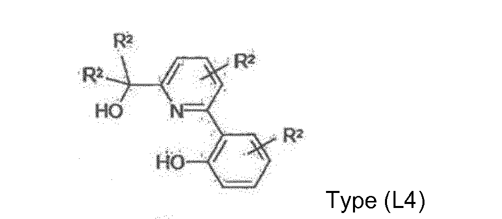 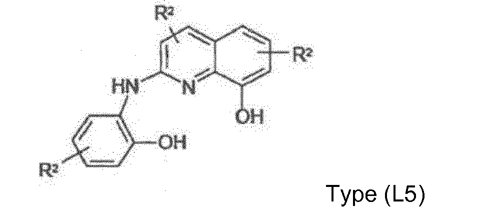 et où
R¹ représente un substituant choisi dans le groupe constitué par :
un atome d'hydrogène, un groupe alkyle ayant de 1 à 20 atomes de carbone, un groupe aryle ou hétéroaryle, un groupe OR, dans lequel R représente un atome d'hydrogène ou un groupe alkyle ayant de 1 à 20 atomes de carbone, un groupe NRR', dans lequel R et R' représentent indépendamment l'un de l'autre un atome d'hydrogène ou un groupe aryle ou alkyle ayant de 1 à 20 atomes de carbone ;
R² représente un substituant choisi dans le groupe constitué par :
un atome d'hydrogène, un groupe alkyle ayant de 1 à 20 atomes de carbone, un groupe aryle ou un groupe hétéroaryle, le groupe aryle ou le groupe hétéroaryle étant respectivement soit lié par une liaison au ligand tridenté soit soudé à ce dernier par deux liaisons C-C, un groupe -COR, -COOR, -COOH, OH, OR, NRR', R et R' représentant indépendamment l'un de l'autre un atome d'hydrogène ou un groupe aryle ou alkyle ayant de 1 à 20 atomes de carbone,
et
R² peut substituer une ou plusieurs fois le cycle aromatique ;
L représente un ligand neutre choisi dans le groupe constitué par :
l'eau, des composés aza-aromatiques, des amines cycliques ou acycliques, des carboxamides cycliques ou acycliques, des esters d'acides carboxyliques cycliques ou acycliques, des cétones cycliques ou acycliques, des cétimines cycliques ou acycliques, des éthers aliphatiques, des alcools aliphatiques ou aromatiques, des sulfoxydes, des organocarbonates cycliques ou acycliques, des nitriles ;
m est égal à 0, 1, 2 ou 3
et
Z⁻ représente un ligand monoanionique choisi dans le groupe constitué par:
des sulfonates, carboxylates, pseudohalogénures, [BF₄]⁻, [PF₆]⁻, [SbF⁶]⁻, le nitrate, l'hydrogénosulfate, le fluorosulfate et le méthylsulfate,
dans la préparation d'isocyanates.

13. Utilisation selon la revendication 11 ou 12, dans laquelle
(O∼N∼O)²⁻ représente un ligand chélateur tridenté, dianionique, coordinant M avec deux atomes d'oxygène et un atome d'azote, qui dérive, par déprotonation de toutes les fonctions OH, de ligands chélateurs neutres choisis dans le groupe constitué par :

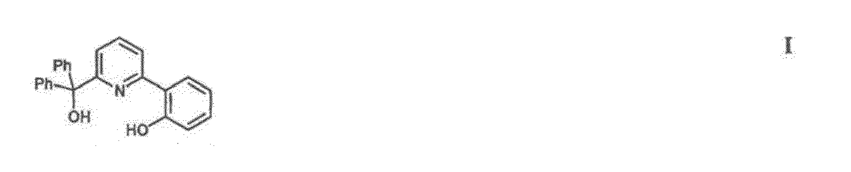

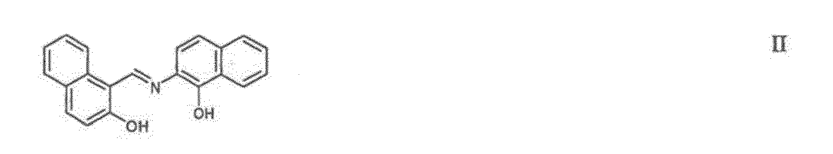

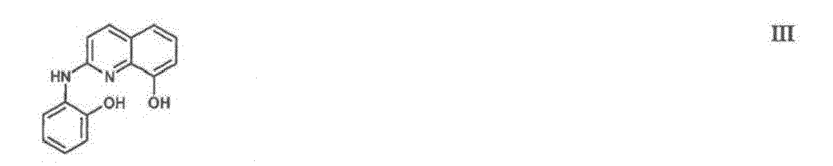

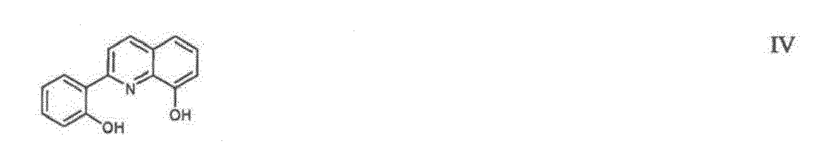

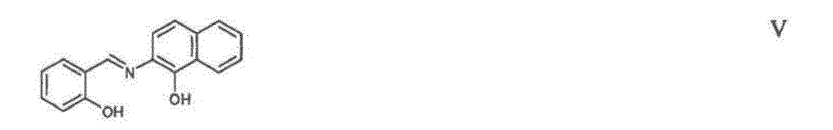

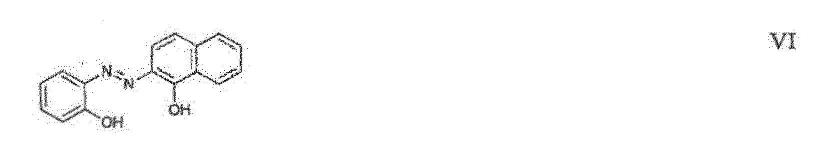

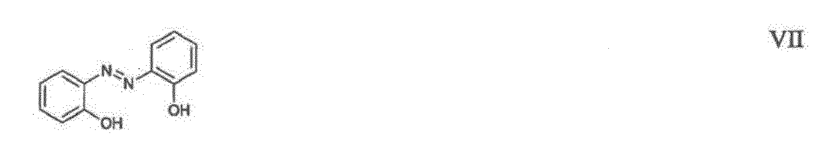

14. Utilisation selon la revendication 11, 12 ou 13, dans laquelle les ligands dianioniques tridentés (O∼N∼O)²⁻ occupent trois sites de coordination méridionaux du cobalt.
